(19) Europäisches Patentamt / European Patent Office / Office européen des brevets

(11) EP 4 679 441 A1

(12) EUROPEAN PATENT APPLICATION

(43) Date of publication:
**14.01.2026 Bulletin 2026/03**

(21) Application number: **25187907.8**

(22) Date of filing: **07.07.2025**

(51) International Patent Classification (IPC):
***G16H 20/17*** *(2018.01)* ***A61B 5/145*** *(2006.01)*
***A61B 5/00*** *(2006.01)* ***G16H 40/63*** *(2018.01)*
***G16H 50/30*** *(2018.01)* *A61M 5/172 (2006.01)*
*A61M 5/142 (2006.01)*

(52) Cooperative Patent Classification (CPC):
**G16H 20/17; A61B 5/14532; A61B 5/4839; G16H 40/63; G16H 50/30;** A61M 5/1723; A61M 2205/3553; G16H 20/60

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **08.07.2024 US 202463668618 P**

(71) Applicant: **Insulet Corporation**
**Acton, MA 01720 (US)**

(72) Inventors:
- **LEE, Joon Bok**
  **Acton, MA, 01720 (US)**
- **ZHENG, Yibin**
  **Hartland, WI, 53029 (US)**
- **CARL, Sam**
  **Waltham, MA, 02453 (US)**
- **O'CONNOR, Jason**
  **Acton, MA, 01720 (US)**

(74) Representative: **Peterreins Schley**
**Patent- und Rechtsanwälte PartG mbB**
**Hermann-Sack-Straße 3**
**80331 München (DE)**

# (54) METHODS TO RAPIDLY ASSESS CHANGES IN MEDICAMENT NEEDS FOR ACCELERATED SHORT-TERM ADAPTIVITY OF AUTOMATED MEDICAMENT DELIVERY SYSTEMS

(57) Exemplary embodiments relate to automated medicament delivery (AMD) devices. Exemplary methods and apparatuses allow the AMD to account for the eventual impact of remaining medicament-on-board (MOB) on reduction in glucose concentrations, and do not incorporate the impact of meals on increases in glucose concentrations. This allows the AMD system to estimate the user's final glucose concentration if the impact of both carbohydrate ingestion and existing MOB are fully realized. Consequently, an AMD system can personalize its behaviors to a particular user more rapidly than if the system were to rely simply on previous medicament delivery and glucose histories. This is especially useful when a user begins using an AMD system with an inaccurate or poorly estimated initial value for total daily medicament (TDM) delivery.

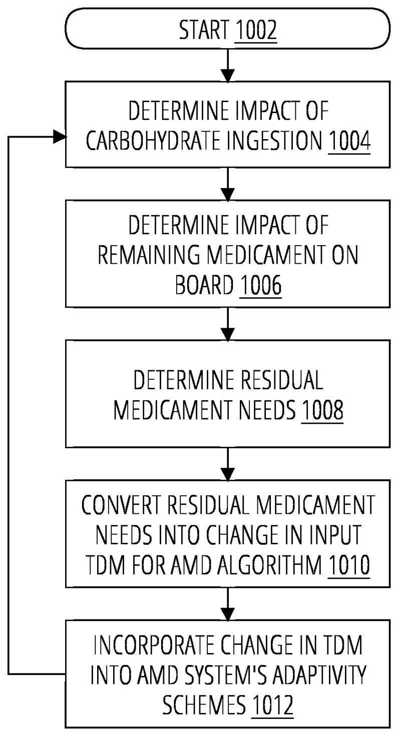


FIG. 10A

EP 4 679 441 A1

## Description

### BACKGROUND

Blood glucose control is the process by which the body maintains a stable level of glucose in the bloodstream, which is crucial for providing energy to the body's cells. The main hormone responsible for blood glucose control is insulin, which is produced by the pancreas.

In people with diabetes, blood glucose control is impaired because the body either doesn't produce enough insulin (Type 1 diabetes) or the body's cells are resistant to insulin (Type 2 diabetes). This leads to high blood glucose levels, which can cause damage to organs and tissues over time.

Managing blood glucose levels is an essential part of diabetes management. This may involve regular monitoring of analyte (such as blood glucose or ketones) levels, medication, dietary changes, and exercise. By carefully managing blood glucose levels, people with diabetes can reduce the risk of complications and lead healthy, active lives.

In order to manage their diabetes, diabetics may use one or more medicaments, such as insulin, glucagon, tirzepatide, glucagon-like peptide (GLP-1), glucose-dependent insulinotropic polypeptide (GIP), a combination of insulin and GLP-1 and/or GIP, and others. Although some examples are described in connection with management of blood glucose, exemplary devices may be suitable for use with other types of medicaments, such as fertility drugs and white blood cell stimulating drugs.

Automated medicament delivery (AMD) systems are designed to improve the glycemic control of people with diabetes. AMD systems typically utilize previous medicament delivery and analyte histories to assess whether the algorithms within the AMD system needs to be modified to improve glucose control outcomes. However, due to the need to avoid modifying the algorithm to compensate for temporary disturbances in insulin needs, such personalization of AMD algorithms are typically executed across longer periods of action. This means that sub-optimal glucose control outcomes may persist for an extended duration during those periods.

### BRIEF SUMMARY

In one aspect, a computer-implemented method may include receiving a total daily medicament value representing an amount of medicament currently being delivered to a user by an automated medicament delivery device on a daily basis, determining the user's ingestion of carbohydrates over a predetermined period of time, determining the user's current remaining medicament-on-board, using the ingestion of carbohydrates and the current remaining medicament-on-board to update the total daily medicament value, and causing the automated medicament delivery device to deliver medicament to the user based on the updated total daily medicament value.

The computer-implemented method may also include calculating an amount of carbohydrates-on-board for the user based on the user's ingestion of carbohydrates in the predetermined period of time, and applying the carbohydrates-on-board when updating the total daily medicament value.

In some embodiments, determining the user's ingestion of carbohydrates over the predetermined period of time may include receiving input from the user includes an estimate of the amount of carbohydrates consumed in the predetermined period of time. Alternatively or in addition, determining the user's ingestion of carbohydrates may include estimating an amount of carbohydrates based on bolus medicament amounts delivered to the user during a meal.

In some embodiments, using the current remaining medicament-on-board to update the total daily medicament value may include determining an impact of the current remaining medicament-on-board by adjusting the current remaining medicament-on-board based on a medicament sensitivity factor.

In some embodiments, using the ingestion of carbohydrates and the current remaining medicament-on-board to update the total daily medicament value may include determining a residual medicament need for the user based on the user's expected current analyte level after an effect of the ingested carbohydrates and medicament-on-board is accounted for. In some embodiments, the residual medicament need may be determined by adjusting the expected current analyte level based on a sensitivity factor.

The computer-implemented method may also include computing the total daily medicament value at predetermined intervals. In some embodiments, the total daily medicament value may be updated only when a confidence interval of a change in the total daily medicament value is determined to be stable.

The techniques described above may be performed separately or in any combination to achieve synergistic effects. Embodiments further include a non-transitory computer-readable medium storing instructions that, when executed by one or more processors, cause the processors to perform any of the methods as described above. Embodiments further include an automated medicament delivery system including one or more processors, and a non-transitory computer-readable medium storing instructions that, when executed the one or more processors, cause the processors to perform the method of any of the above-described methods.

Other technical features may be readily apparent to one skilled in the art from the following figures, descriptions, and claims.

## BRIEF DESCRIPTION OF THE SEVERAL VIEWS OF THE DRAWINGS

To easily identify the discussion of any particular element or act, the most significant digit or digits in a reference number refer to the figure number in which that element is first introduced.

FIG. 1 depicts an exemplary medicament delivery system 100 in accordance with one embodiment.

FIG. 2A depicts a system that integrates an automated insulin delivery device and a continuous glucose monitor in accordance with one embodiment.

FIG. 2B depicts the system of FIG. 2A in greater detail.

FIG. 3A depicts a standalone continuous glucose monitor in accordance with one embodiment.

FIG. 3B depicts the monitor of FIG. 3A in more detail.

FIG. 3C depicts the monitor of FIG. 3A in more detail.

FIG. 4 is a flow chart that provides an overview of a system initialization and operation procedure for a medicament delivery system 100 in accordance with one embodiment.

FIG. 5 depicts the component detection process of FIG. 4 in more detail.

FIG. 6 depicts the initialization process of FIG. 4 in more detail.

FIG. 7A depicts the delivery process of FIG. 4 in more detail

FIG. 7B depicts the event detection process of FIG. 4 in more detail

FIG. 7C depicts the notification process of FIG. 4 in more detail.

FIG. 8 depicts the short-term adaptation process of FIG. 4 in more detail.

FIG. 9 depicts the long-term adaptation process of FIG. 4 in more detail.

FIG. 10A illustrates exemplary logic in accordance with one embodiment.

FIG. 10B illustrates exemplary logic in accordance with one embodiment.

FIG. 10C illustrates exemplary logic in accordance with one embodiment.

FIG. 10D illustrates exemplary logic in accordance with one embodiment.

## DETAILED DESCRIPTION

In an algorithm for an automated medicament delivery (AMD) device, the user's current glucose concentrations and existing medicament-on-board (MOB) may be used to estimate the extent of acceptable additional medicament delivery. However, using only these parameters does not allow the AMD system to directly account for the eventual impact of remaining MOB on reduction in glucose concentrations, and does not incorporate the impact of meals on increases in glucose concentrations.

In the exemplary embodiment discussed below, the determination of whether the user has currently received sufficient insulin may be made by incorporating these two factors (impact of remaining MOB and impact of meals) directly into the user's glucose concentrations, to estimate the user's final glucose concentration if the impact of both carbohydrate ingestion and existing MOB are fully realized.

Consequently, an AMD system can personalize its behaviors to a particular user more rapidly than if the system were to rely simply on previous medicament delivery and glucose histories. This is especially useful when a user begins using an AMD system with an inaccurate or poorly estimated value for TDM. Because exemplary embodiments allow the user to receive a more accurate amount of medicament that is better tuned to their needs, the user may experience a reduced chance for hypo- or hyper-glycemic events. Furthermore, the user may avoid the need for some bolus doses they would have otherwise triggered, which can reduce the power consumption of the automated medicament delivery device 108. This may allow for a lighter device with a smaller battery, or a more long-lasting device.

Several different embodiments will be described below. Each provides advantages, and it is contemplated that any embodiment may be employed individually (e.g., in connection with a control algorithm and system as described in connection with FIG. 1 - FIG. 3C), or in any combination to yield synergistic improvements.

*A Note on Data Privacy*

Some embodiments described herein make use of training data or metrics that may include information voluntarily provided by one or more users. In such embodiments, data privacy may be protected in a number of ways.

For example, the user may be required to opt in to any data collection before user data is collected or used. The user may also be provided with the opportunity to opt out of any data collection. Before opting in to data collection, the user may be provided with a description of the ways in which the data will be used, how long the data will be retained, and the safeguards that are in place to protect the data from disclosure.

Any information identifying the user from which the data was collected may be purged or disassociated from the data. In the event that any identifying information needs to be retained (e.g., to meet regulatory requirements), the user may be informed of the collection of the identifying information, the uses that will be made of the identifying information, and the amount of time that the identifying information will be retained. Information specifically identifying the user may be removed and may be replaced with, for example, a generic identification number or other non-specific form of identification.

Once collected, the data may be stored in a secure data storage location that includes safeguards to prevent unauthorized access to the data. The data may be stored in an encrypted format. Identifying information and/or non-identifying information may be purged from the data storage after a predetermined period of time.

Although particular privacy protection techniques are described herein for purposes of illustration, one of ordinary skill in the art will recognize that privacy protected in other manners as well. Further details regarding data privacy are discussed below in the section describing network embodiments.

Assuming a user's privacy conditions are met, exemplary embodiments may be deployed in a wide variety of messaging systems, including messaging in a social network or on a mobile device (e.g., through a messaging client application or via short message service), among other possibilities. An overview of exemplary logic and processes for engaging in synchronous video conversation in a messaging system is next provided.

Exemplary Embodiments

As an aid to understanding, a series of examples will first be presented before detailed descriptions of the underlying implementations are described. It is noted that these examples are intended to be illustrative only and that the present invention is not limited to the embodiments shown.

Reference is now made to the drawings, wherein like reference numerals are used to refer to like elements throughout. In the following description, for purposes of explanation, numerous specific details are set forth in order to provide a thorough understanding thereof. However, the novel embodiments can be practiced without these specific details. In other instances, well known structures and devices are shown in block diagram form in order to facilitate a description thereof. The intention is to cover all modifications, equivalents, and alternatives consistent with the claimed subject matter.

Next, a basic medicament delivery system 100 will be described. As shown in FIG. 1, the medicament delivery system 100 may include an automated medicament delivery device 108, an analyte sensor 110, and a control device 102.

An automated medicament delivery device 108 is a small device that delivers a medicament, such as insulin to the body in a controlled and continuous manner. An automated medicament delivery device 108 allows for more precise and flexible medicament delivery compared to injections, as the user can adjust their basal rate and bolus doses to match their individual medicament needs. It can also provide greater convenience and discretion in daily life.

An exemplary automated medicament delivery device 108 may include a medicament pump 122 with a reservoir that holds the insulin and a plunger inserted into the reservoir. Other exemplary embodiments may be plunger-less. Reciprocating pumps and pumps using a pre-filled cartridge are also contemplated. Embodiments are not limited to these types of delivery mechanisms; any suitable delivery mechanism may be employed.

In the depicted type of pump, medicament is filled into the reservoir of the pump, either directly with a syringe or through a special filling device. In some automated medicament delivery devices 108, the reservoir is provided to the user already filled with medicament. The plunger can be withdrawn from the reservoir to allow the reservoir to be filled, or driven into the reservoir to dispense the medicament through a needle. A small motor or other actuator may drive the plunger forward or backwards (or the plunger may be driven under fluid pressure and/or an action of the user), for instance by turning a lead screw that moves a nut attached to the plunger.

Computing hardware and/or software may control the delivery of medicament. For example, a processor 120 may execute a control algorithm, such as the model predictive control (MPC) algorithm described below. The control algorithm may be embodied as instructions stored in a memory 118, which may be a non-transitory computer-readable medium such as flash memory, a hard drive or solid state drive, removable media, etc. The control algorithm may be stored and run on the pump itself, or on another device that communicates with the pump.

The control algorithm may perform several functions, such as calculating basal rates and bolus doses and delivering such doses. The basal rate is the amount of medicament that is delivered to address the body's normal metabolic needs outside of meals to keep the blood glucose levels stable, or in a euglycemic range. A basal rate may be set based on a user's individual medicament needs. Around the time the user is eating (e.g., when the user is about to eat), they can use the pump to deliver a bolus dose of medicament. A bolus dose is an amount of medicament that is delivered all at once to address consumption of foods such as carbohydrates. In an exemplary hybrid closed-loop or other automated medicament delivery device 108, the user inputs the amount of carbohydrates consumed or intended to be consumed and the algorithm determines the amount of medicament needed based at least on the carbohydrate amount and the current, historical, and/or predicted blood glucose level.

The control algorithm may also generate alerts when a problem arises. For instance, the automated medicament delivery device 108 can alert the user to a condition such as a low battery, an occlusion in the fluid line of the of the automated medicament delivery device 108, or if the user forgets to take a bolus dose.

The status of the automated medicament delivery device 108 and any generated alerts may be displayed, either on a small screen on the automated medicament delivery device 108 itself or on a display of a separate control device 102. The displayed information can include the current basal rate, medicament on board, current glucose level, and/or other important information. The control device 102 may also allow a user to manually enter their blood glucose as measured by a fingerstick glucose meter. Alternatively, the control device 102 may display blood glucose levels as measured with an analyte sensor 110.

The automated medicament delivery device 108 can send information to, or receive information from, the control device 102 and/or analyte sensor 110 using a transmitter/receiver 104. The transmitter/receiver 104 may enable wireless communication; for instance, it may be a BLUETOOTH(R) or BLUETOOTH Low Energy (LE) radio, or a similar type of device. The control device 102 may include a corresponding transmitter/receiver 124, as well as a memory 126 and a processor 128.

An exemplary analyte sensor 110 is a small device that tracks the level of an analyte, such as glucose or ketones, in interstitial fluid (fluid between the body's cells) continuously throughout the day and night. The analyte sensor 110 may provide data to the automated medicament delivery device 108 to allow the automated medicament delivery device 108 to adjust the amount of insulin being delivered. An analyte sensor 110 such as a continuous glucose monitor provides a more complete picture of glucose trends than traditional fingerstick glucose monitoring, as it provides continuous glucose data and alerts. It can help people with diabetes to manage their glucose levels more effectively and improve their overall health and quality of life.

The analyte sensor 110 may include a transmitter/receiver 106 similar to the transmitter/receiver 104 of the automated medicament delivery device 108. The analyte sensor 110 may also include a memory 114 and a processor 116.

The analyte sensor 110 further includes a sensor 112, such as a glucose sensor or a ketone sensor, for example. The sensor 112 is inserted just under the skin, usually on the abdomen, arm, or thigh. The sensor 112 may have a small filament that is inserted into the interstitial fluid and measures the level of certain chemicals in the user's blood.

For example, a glucose sensor is a device used to measure the concentration of glucose in a person's blood or interstitial fluid. There are several types of glucose sensors, but most work by using an enzyme called glucose oxidase to catalyze the reaction between glucose and oxygen.

When glucose oxidase reacts with glucose, it produces gluconic acid and hydrogen peroxide. The amount of hydrogen peroxide produced is proportional to the amount of glucose present. The glucose sensor measures the amount of hydrogen peroxide and uses it to calculate the concentration of glucose in the blood.

A sensor 112 of this type typically consists of one or more small electrodes that are coated with the enzyme glucose oxidase. The electrodes are inserted under the skin and are connected to a small transmitter that sends the glucose readings to a receiver in the analyte sensor 110.

Another type of sensor 112 is a ketone sensor. A ketone sensor works by measuring the concentration of ketones, which are produced when the body breaks down fat for energy.

A ketone sensor is similar to a glucose sensor in that it uses an enzyme (e.g., beta-hydroxybutyrate dehydrogenase) to detect ketone levels. This enzyme catalyzes the reaction between beta-hydroxybutyrate, a type of ketone, and a coenzyme called NAD+. The reaction produces NADH and acetoacetate, another type of ketone. The ketone sensor measures the amount of NADH produced by the reaction and uses it to calculate the concentration of beta-hydroxybutyrate in the blood. This gives an indication of the level of ketosis in the body.

The sensor 112 is typically good for several days' or weeks' use, and the user can monitor their glucose and/or ketone levels in real-time using the control device 102. The sensor 112 measures the user's glucose and/or ketone level every few minutes and sends this data to the transmitter/receiver 106. The transmitter/receiver 106 may then send this data wirelessly to the transmitter/receiver 104 of the automated medicament delivery device 108 and/or to the transmitter/receiver 124 of the control device 102. In some embodiments where the automated medicament delivery device 108 and analyte sensor 110 are integrated or co-located, a physical (e.g., wired) connection may be provided to allow the automated medicament delivery device 108 and analyte sensor 110 to communicate.

The control device 102 may collect and analyze the glucose and/or ketone data, displaying it as a graph or number. It also provides alerts if the glucose level and/or ketone level goes too high or too low, helping the user to take appropriate action to manage their glucose and/or ketone levels. The data can further be used to identify trends and patterns in glucose and/or ketone levels over time, helping the user to make informed decisions about diet, exercise, medication, and other factors that affect their glucose and/or ketone levels.

The automated medicament delivery device 108 and analyte sensor 110 may be integrated together in a single device, as shown in FIG. 2A and FIG. 2B. In this example, the device includes at least one housing 202 that encompasses both the automated medicament delivery device 108 and the analyte sensor 110.

The automated medicament delivery devices 108 and analyte sensor 110 may not have the same lifespans. For example, the reservoir or cartridge of the automated medicament delivery device 108 may only store enough insulin for a few days, while the sensor 112 of the analyte sensor 110 may remain useful for several more days.

Accordingly, in some embodiments the analyte sensor 110 may be provided in its own analyte sensor housing 302, as shown in FIG. 3A - FIG. 3C. In such embodiments, separate housings may encompass the automated medicament delivery device 108 and the analyte sensor housing 302, and such housings may be positioned adjacent to each other, e.g., on a tray or a cradle or another housing, thereby allowing the automated medicament delivery device 108 to be separately replaced.

Next, a glucose control algorithm will be described. The glucose control algorithm described below represents a basic algorithm that may be further modified using the techniques described in the remainder of the application.

## Control Architecture

The control algorithm may employ a modular design in which core functionality is separated from dependent functionality. Dependent functionality includes functionality that is implementation-specific to the current environment. This includes software abstraction for the analyte sensor 110. The dependent functionality also includes software services which interface with implementation-specific features that affect inputs or outputs to the algorithm, such as the Activity Mode (see below).

An interface may be responsible for managing algorithm initialization and upload of insulin history to a history buffer, managing the algorithm's state and data variables, and maintaining cycle-to-cycle data needed by the algorithm. For this purpose, an analyte sensor interface may retrieve data such as blood glucose and/or ketone measurements from the analyte sensor 110.

The interface may also send commands to the automated medicament delivery device 108 to deliver micro-bolus outputs as calculated by the algorithm. This transmission of data may occur wirelessly or over a bus of the automated medicament delivery device 108. The transmission may occur at regular intervals (e.g., every 1 minute, every 5 minutes, etc.).

The algorithm's core functionality may include a set of procedures that take an estimated glucose value (EGV) as input and compute a recommended insulin dose (in the example of an automated insulin delivery device; other devices employing different sensors and/or medicaments may also be used and the algorithm adjusted accordingly). These layers also include a wide range of safety constraints and edge case handling routines to ensure robust and safe behavior of the algorithm.

## System Parameters

The control algorithm relies on a wide range of parameters and features to operate effectively to calculate its recommended medicament dose. Many parameters remain fixed or are derived from user adjustable inputs. These variable inputs that impact algorithm parameters may include glycemic setpoint, total daily medicament, whether Activity Mode is activated, and user-requested meal and correction boluses. In some embodiments, total daily medicament may be input once, for the first day of medicament delivery, or initially calculated from the user's basal profile. In other embodiments, total daily medicament maybe derived from other parameters without requiring user input.

## Glycemic Setpoint

A glycemic setpoint is a blood glucose target between 90 and 150 mg/dL that the algorithm uses as a baseline. The glycemic setpoint is a concept used to describe the blood glucose level that the body strives to maintain during normal activity. It is similar to the idea of a "set point" for body weight or temperature, which the body works to maintain within a certain range.

In healthy individuals without diabetes, the body's glycemic level is typically around 80-120 mg/dL (4.4-6.7 mmol/L) when fasting and up to 180 mg/dL (7.8 mmol/L) or higher after a meal. The body maintains euglycemia through a complex interplay of hormones, including insulin and glucagon, which help to regulate glucose production and uptake in the liver and muscles.

In people with diabetes, the glycemic level can be altered due to a range of factors, including insulin resistance, impaired insulin production, and lifestyle factors such as diet and exercise. Imbalanced factors can lead to chronically high blood glucose levels (hyperglycemia) or low blood glucose levels (hypoglycemia), both of which can have negative health consequences.

Managing blood glucose levels through medication, dietary changes, and lifestyle modifications can help to bring blood glucose levels closer to the body's natural glycemic setpoint or euglycemic range, reducing the risk of complications and improving overall health.

The control algorithm supports the use of multiple target glucose values set by the user, e.g., 90-150 mg/dL in 10 mg/dL increments.

## Total Daily Medicament

Total daily medicament (TDM) refers to the amount of insulin or another medicament a person with diabetes needs to take each day to maintain normal blood glucose levels. This includes both basal insulin, which provides a steady background level of insulin, and bolus insulin, which is taken before meals to cover the rise in blood glucose that occurs after eating.

The total daily medicament dose can vary greatly depending on a person's individual needs, including factors such as body weight, level of physical activity, diet, stress, and insulin sensitivity. For women, it can also vary based on period of a menstrual cycle or stage of a pregnancy. It is typically calculated based on a person's insulin-to-carbohydrate ratio (ICR) and insulin sensitivity factor (ISF), which are determined through blood glucose monitoring.

In the control algorithm, TDM may be user-adjustable for the first 48 hours of use and afterwards may be dependent on the user's medicament delivery history.

## Activity Mode Activation

Activity Mode activation refers to whether the user activated or did not activate a mode that may be referred to as "Activity Mode." The Activity Mode temporarily changes parameters that are passed to the algorithm to make insulin delivery less aggressive in order to protect against hypoglycemic events, for example, during periods of inactivity. Triggering Activity Mode temporarily sets insulin delivery to a lower value to prevent entering hypoglycemic range during activities which can lower blood glucose level.

## User Requested Meal and Correction Boluses

User requested meal and correction boluses refer to the size of a bolus that can be given parallel to algorithm delivery to accompany ingestion of any meals.

## System Initialization

Achieving optimal blood glucose control through insulin therapy requires careful monitoring and adjustment of insulin doses based on individual needs and changing circumstances. People with diabetes who use insulin may work with a healthcare team to develop an individualized insulin regimen that meets their needs and helps them achieve their blood glucose goals. During algorithm initialization, the user's health care provider may assist the user to program basal rates, glucose targets and bolus calculator settings, although in some embodiments it may not be necessary to receive assistance from a health care provider.

## Ongoing Blood Glucose Control

The exemplary control algorithm described below is a model predictive control (MPC) algorithm. Model Predictive Control (MPC) is an advanced computational control approach that uses a mathematical model of a system to make predictions of future system behavior and optimize control actions.

The MPC algorithm uses a dynamic model of the system (in this case, the system may represent the insulin-glucose dynamics of the user) and an optimization algorithm to predict the future behavior of the system and find an optimal control action. For instance, the algorithm may predict the user's future blood glucose in response to a proposed set of insulin doses.

The algorithm optimizes the control input by minimizing a cost function over a finite time horizon, subject to constraints on the system variables and control input. A cost function, also known as an objective function or loss function, is a mathematical function that quantifies the error or discrepancy between the predicted output of a model and the actual or desired output. In exemplary embodiments, the cost function may assess the cost of predicted glucose deviations (from a setpoint) and insulin delivery deviations (from the user's typical basal rate); several iterations may be run through and the one with the lowest cost may be chosen. One purpose of a cost function is to measure how well a model is performing and to guide the learning or optimization process towards finding the optimal set of parameters. By minimizing or maximizing the cost function, the model can adjust its parameters to achieve the desired outcome. Examples of common types of cost functions include (but are not limited to) Mean Squared Error (MSE), Binary Cross-Entropy, Categorical Cross-Entropy, Hinge Loss, and Kullback-Leibler Divergence.

The control action is applied over the finite time horizon, after which the optimization is repeated using the updated system state. This process is repeated iteratively, resulting in a sequence of optimal control actions that are applied to the system over time.

MPC has many advantages, including the ability to handle complex multivariable systems with constraints, the ability to incorporate disturbances and uncertainties into the control strategy, and the ability to handle non-linear dynamics.

The model within the medicament delivery system 100 may predict the evolution of glucose values over a prediction horizon consisting of a certain number (e.g., 6, 12, 24, etc.) time-steps of relatively short (e.g., 1, 3, 5, 10, etc. minutes) duration. This results in a predicted glucose over a prediction horizon (e.g., a 30-, 60-, 120-, etc minute horizon). The model takes in estimated glucose values (EGV) from the analyte sensor 110 through Bluetooth Low Energy (BLE) and calculates small doses of insulin accordingly for the automated medicament delivery device 108 to deliver.

In obtaining blood glucose measurements, the algorithm may apply rate of change filters. The rate of change filters factor in the real change in analyte sensor values that can occur in the human body over a period of time due to physiology. They may also limit glucose predictions of the system to be no more than a threshold value.

The algorithm may also maintain the analyte sensor 110 state based upon analyte sensor 110 values received to determine the output of the algorithm. It also can provide estimates of missing analyte sensor 110 data handling and analyte sensor predictions. The missing data handling provides logic that controls the automated medicament delivery device 108, allowing the automated medicament delivery device 108 to behave robustly when the analyte sensor 110 is not available due to communication interruptions between the automated medicament delivery device 108 and the analyte sensor 110.

The model may penalize both deviations of predicted glucose values from the setpoint as well as deviations of the recommended insulin from a determined insulin basal rate. At each time-step, the algorithm determines an optimal set of insulin doses (micro-boluses) over a control horizon consisting of a certain number of timesteps that minimizes the cost of these two deviations. MPC is based on real-time numerical optimization and thus allows for a large degree of flexibility in achieving the control objective. Based on the optimal control action identified by the algorithm over the time horizon (e.g., an insulin dose that minimizes the cost function over the time horizon), a medicament dosage amount may be determined and a medicament dose command may be transmitted to the medicament pump 122.

At each time-step, the medicament dose command from the algorithm is bounded by multiple safety constraints on medicament delivery, as managed by algorithm constants and safety limits.

These constraints include a dynamic constraint based on a medicament-on-board (MOB) model incorporated into the optimization algorithm. Medicament on board (MOB) refers to the amount of medicament, such as insulin, that has not yet acted in a user's body after their last medicament dose. This is typically measured in units of medicament and is used to help people with diabetes manage their blood sugar levels.

When the user injects insulin or uses an insulin pump, the insulin begins working to lower their blood sugar. However, it takes some time for the insulin to take effect, and even after it does, it continues to work for several hours. This means that there may be a period of time during which the user has both the insulin from their previous dose and the insulin from their current dose working in their body.

Knowing MOB can be helpful for managing blood sugar levels, as it can give an idea of how much insulin is still working in a user's body and how it may be affecting the user's blood sugar. Some automated medicament delivery devices and analyte sensors can estimate MOB automatically based on the amount and timing of medicament doses.

Optimal post-prandial control may require the user to give meal boluses (as is required in conventional pump therapy), but normal operation of the MPC algorithm may compensate for missed meal boluses and mitigate prolonged severe hyperglycemia. During operation, logic may detect rescue carbohydrate ingestion and apply safe upper limits to micro bolus delivery. Rescue carbs handling logic may detect rescue carbohydrate ingestion based on readings from the analyte sensor 110 and/or from other external sensors.

System Changes

The automated medicament delivery device 108 may be used for a certain period of time and then replaced by a new automated medicament delivery device 108. During such a system change, the user's medicament data (including TDM) is saved to a buffer (e.g., stored on the control device 102 and/or a remote server). During every AMD system change, before deactivation of the medicament delivery system 100, the data from the buffer is transferred to an app on the control device 102, which updates its previously saved data, storing new data to be consumed by the MPC Algorithm. Over several medicament delivery system 100 changes, the algorithm adapts to the user's TDM needs.

The algorithm estimates the Medicament on Board (MOB) from automated medicament delivery and provides this to the app on the control device 102. The Algorithm MOB, along with recent boluses recorded by the app, are utilized to calculate total MOB. The total MOB is displayed to the user on the control device 102.

FIG. 4 illustrates an example routine for controlling an automated medicament delivery device 108 with limited or no user input. Although the example routine depicts a particular sequence of operations, the sequence may be altered without departing from the scope of the present disclosure. For example, some of the operations depicted may be performed in parallel or in a different sequence that does not materially affect the function of the routine. In other examples, different components of an example device or system that implements the routine may perform functions at substantially the same time or in a specific sequence.

Note that the blocks depicted in FIG. 4 reference general processes that may be performed in connection with the deployment and operation of an AMD. Existing processes are known for some of these steps, although exemplary processes are expressly described for each process in connection with FIG. 5 - FIG. 9. It is contemplated that all of the novel processes may be used in the overall processes, or a mix of old and new processes may be employed. Thus, the present disclosure is not limited to the situation where all of the processes of FIG. 5 - FIG. 9 are used in the appropriate blocks in FIG. 4. Nonetheless, it is contemplated that all of these processes could be used together in an exemplary embodiment.

Processing begins at start block 402. Start block 402 may be triggered, for example, upon an initial startup of a new AMD system. The initial startup may trigger provisioning logic that causes the AMD system to perform a first-time setup procedure.

Processing may then proceed to component detection process 500, where the AMD system performs component detection. As previously discussed, AMD systems typically incorporate an analyte sensor 110 and an automated medicament delivery device 108 including a medicament pump 122 controlled by an AMD algorithm. The user interface that allows the user to assess the status of the system and/or interact with the system may be part of the automated medicament delivery device 108, in case of durable components, or it may be a separate component such as the control device 102.

A simplified, no-setting AMD system may automatically detect an advertising analyte sensor 110 via their Bluetooth signal, and pair with the analyte sensor 110 without requiring any user input. Optionally, the AMD may ask for confirmation that the sensor and AMD system should be paired. Moreover, the automated medicament delivery device 108 may automatically detect a non-durable medicament pump 122 that has been "primed" and in range, without the user needing to enter any identifying parameters. In a similar manner, a new AMD system may automatically establish a connection with an existing and operational AMD system. Security measures, such as physical on-device confirmation display requirements, may be employed. The component detection process is described in more detail in connection with FIG. 5.

According to some examples, the method may proceed with initialization at initialization process 600. The initialization process 600 sets system parameters used to determine amounts and/or timings of medicament doses to initial values. These values may be derived from a total daily medicament value. The initial total daily medicament value may be determined, wholly or in part, based on population-based default values. A predicted-safe value may be used for the initial total daily medicament value. The predicted-safe value may be based on the population-based default value and the capability of the automated medicament delivery device 108 to compensate for over- or under-delivery of medicament. The initialization process 600 is described in more detail in connection with FIG. 6.

According to some examples, the method includes delivering at delivery process 700. As noted above, the process of delivering medicament to a user may be subject to several safety constraints (e.g., a medicament-on-board constraint, a maximum cycle dose constraint, system upper bounds, etc). Values for the safety constraints may be initially set based on the initial total daily medicament value determined in the initialization process 600. The delivery process is described in more detail in connection with FIG. 7A.

According to some examples, the method includes event detection at event detection process 406. The event detection process may use data available to the automated medicament delivery device 108 and/or control device 102 to determine whether an analyte control event is occurring. For example, in the case of controlling a user's blood glucose, events such as meal ingestion or exercise may affect the ability of the automated medicament delivery device 108 to control the user's blood glucose. Detection of such an event may cause the automated medicament delivery device 108 to enter into a different operating state or mode, or to otherwise adjust medicament delivery parameters. Events may be detected, for example, by reviewing analyte trends from analyte values measured by the analyte sensor 110 and/or by reviewing external sensor data from devices such as the control device 102, a fitness tracker, global positioning device, etc. The event detection process is described in more detail in connection with FIG. 7B.

According to some examples, the method includes a notification process at notification process 408. When the user experiences an analyte control event (e.g., an instance of hypo- or hyper-glycemia), a notification may be generated to inform the user of the event. Depending on how the user interacts with the notification, the frequency of notifications may be adjusted. For example, if the user generally acknowledges notifications quickly, the rate or frequency of notifications may be decreased so as to not interfere with the user's daily activities. On the other hand, if the user fails to respond to one or more notifications, the frequency of notifications may be increased, the notification may be escalated (e.g., by adding sound or vibration), and/or the notification may be escalated to third parties such as authorized emergency contacts. The notification process is described in more detail in connection with FIG. 7C.

According to some examples, the method includes an adaptation process at block 404. The adaptation process adjusts the initial values for total daily medicament and other parameters over time, as additional analyte measurement data becomes available. The adaptation process may include a short term adaptation process 800 (discussed in more detail in connection with FIG. 8) and a long term adaptation process 900 (discussed in more detail in connection with FIG. 9).

FIG. 5 depicts the component detection process 500 of FIG. 4 in more detail. Although the example component detection process 500 depicts a particular sequence of operations, the sequence may be altered without departing from the scope of the present disclosure. For example, some of the operations depicted may be performed in parallel or in a different sequence that does not materially affect the function of the component detection process 500. In other examples, different components of an example device or system that implements the component detection process 500 may perform functions at substantially the same time or in a specific sequence.

According to some examples, the method includes deploying an analyte sensor 110 at block 502. The analyte sensor 110 may be a standalone device that is deployed separately from an automated medicament delivery device 108. The analyte sensor 110 may be placed on a user's body.

The analyte sensor 110 may be configured to broadcast measurements of an analyte at regular intervals to connected devices (e.g., the automated medicament delivery device 108). In order to connect to these devices, according to some examples, the analyte sensor may broadcast a wireless (e.g., Bluetooth) signal at block 504. The wireless signal may be an initiation of a device-specific handshake procedure.

According to some examples, the method includes automated medicament delivery device 108 startup at block 506. For example, the user may fill the automated medicament delivery device 108 with medicament (or medicament may be pre-loaded in the automated medicament delivery device 108), and then the user may place the automated medicament delivery device 108 on their body. The automated medicament delivery device 108 may detect that it has been placed on the body (e.g., based on a sensor output). Upon detecting its placement on the body, the automated medicament delivery device 108 may begin scanning for a broadcast wireless signal, such as the aforementioned handshake procedure.

According to some examples, the automated medicament delivery device 108 may receive the wireless signal at block 508. At block 510, the automated medicament delivery device 108 may optionally cause pairing information (e.g., a device identifier of the analyte sensor 110 attempting to pair with the automated medicament delivery device 108) on a display of the automated medicament delivery device 108 or the control device 102.

According to some examples, automated medicament delivery device 108 may optionally request user confirmation that the analyte sensor 110 should be paired at block 512. For example, a display of the automated medicament delivery device 108 or the control device 102 may display an indication that the analyte sensor 110 is attempting to pair, and asking that the user accept the pairing (e.g., by tapping an on-screen confirmation button). In some embodiments, the user may confirm the pairing by not canceling the pairing request within a predetermined period of time (e.g., but not tapping an on-screen cancelation button).

According to some examples, analyte sensor 110 may be automatically paired with the automated medicament delivery device 108 without any (further) user interaction at block 514. For example, the automated medicament delivery device 108 and analyte sensor 110 may complete the aforementioned handshake process to establish a communication connection.

In some embodiments, the component detection process 500 may terminate at this stage, or (through block 524) may revert back to block 502 so that the old analyte sensor 110 can be removed and a new analyte sensor 110 can be paired with the automated medicament delivery device 108 with limited or no user interaction.

In other embodiments, the component detection process 500 may also allow the medicament pump 122 of the automated medicament delivery device 108 to be removed and replaced, and for the automated medicament delivery device 108 to establish communication with the new medicament pump 122. To this end, at block 516 an existing non-durable (i.e., replaceable) pump may be removed.

When a new pump is primed (e.g., filled with a medicament and placed in a configuration such that it is capable of dispensing the medicament), then the new pump may begin broadcasting a wireless handshake signal (similar to the one previously described in connection with the analyte sensor 110) at block 518.

The automated medicament delivery device 108 may detect the wireless signal at block 520 and, in a manner similar to blocks 510-514 above, may automatically pair with the replacement pump at block 522.

Although the above described procedure relates to pairing an analyte sensor 110 with an automated medicament delivery device 108, the same technique may be used to pair other devices with the automated medicament delivery device 108 without requiring user input. For example, the automated medicament delivery device 108 may be paired with a heart rate sensor, accelerometer, the control device 102, a wearable fitness or medical tracker, etc.

Once the medicament delivery system 100 is connected across all relevant components, the medicament delivery system 100 may also initiate its insulin deliveries without any user input (or with only limited user input as described below).

Typical medicament delivery systems 100 operate based on a set of parameters, such as the user's analyte (e.g., glucose) control target, total medicament (e.g., insulin) needs, and/or bolus medicament needs. However, a robustly designed medicament delivery system 100 can start medicament delivery without prior user input. This may be achieved by a thorough risk assessment and various constraints to allow the system to deliver a reduced insulin delivery amount, which when paired with analyte feedback will protect users from over-delivery. Previous medicament delivery and/or analyte history from devices that are connected may be used to improve this initialization.

A reasonable, safe assumption of initial parameters, such as basal medicament needs, target analyte concentrations, and general system settings, can be executed to cover a great majority of each user's use patterns. This may be based on typical population metrics, with reasonable statistical analyses of standard deviations and the AMD algorithm's robustness designs.

FIG. 6 illustrates an example initialization process 600 in more detail. Although the example initialization process 600 depicts a particular sequence of operations, the sequence may be altered without departing from the scope of the present disclosure. For example, some of the operations depicted may be performed in parallel or in a different sequence that does not materially affect the function of the initialization process 600. In other examples, different components of an example device or system that implements the initialization process 600 may perform functions at substantially the same time or in a specific sequence.

According to some examples, the method includes estimating total daily medicament needs based on population-based default values at block 602.

The most fundamental clinical parameter utilized by people with insulin-dependent diabetes is total daily medicament, or TDM. This TDM value may be further incorporated into calculations for a wide range of derivative clinical parameters, such as basal insulin profiles, insulin to carbohydrate ratio, correction factor, and others. Consequently, TDM values are available for most, if not all, users who may transition into AMD systems, even if those users do not yet utilize the derivative parameters, such as is the case for multiple daily injection users.

When transitioning to a no- or limited- user-input paradigm, an initial TDM value must be determined that protects against over- or under- delivery of medicament. If too much medicament is provided to a diabetic user, the user may experience hypoglycemia as their blood glucose level drops to dangerous levels. If too little medicament is provided, the user may experience hyperglycemia as their blood glucose level rises to dangerous levels.

One insight useful in setting the initial TDM value is that, when paired with an analyte sensor 110 that retrieves frequent measurements of the user's analyte levels (e.g., blood glucose or ketones), a suitable automated medicament delivery device 108 can make continuous adjustments to the amount of medicament delivered, and can therefore provide robust protection against medicament over- or under delivery (up to a certain degree). The degree to which the automated medicament delivery device 108 and analyte sensor 110 can compensate for an initial over- or under- estimate of TDM can inform the initial selection of the TDM value.

More specifically, AMD systems typically deliver insulin in smaller increments, as an AMD algorithm often incorporates continuous medicament measurements from an analyte sensor 110 such as a continuous glucose monitor (CGM). Therefore, it is feasible for an AMD system to potentially be initialized with a relatively higher insulin delivery rate, and allow the AMD system's feedback mechanisms to reduce delivery as needed. In converse, there is a significantly greater risk in bolus deliveries, particularly manual bolus deliveries, due to the large quantity of any one-time delivery, magnifying the impact of erroneous initializations. Therefore, a safe initialized system may limit the initial delivery over time.

For example, the general population's average total insulin needs may be approximately 40U. A reasonable starting estimate for AMD systems may then be a total insulin value below that amount, assuming the system can adapt rapidly to the user's insulin needs based on analyte outcomes. In one embodiment, this value may be set to 20U, assuming that the AMD system may be able to compensate for reduced insulin need for users with up to 50% less than this value (such as 10U):

$$TDI_{est}(1) = 20U$$

In one example embodiment, an AMD system may initialize its parameters with an assumed safe initial estimate of the user's TDM without any inputs from the user. However, such an AMD system will generally behave in a conservative manner to minimize risk of over delivery of insulin and resulting hypoglycemia. This may be embodied as 1) a low initial estimate of the default TDM value, 2) stronger limitation by the system's safety constraints, and 3) a slow rate of change of the TDM parameter.

Optionally, the user may specify an initial TDM value at block 604. Although next generation AMD systems may be designed to reduce or eliminate the inputs needed for users to both initialize and maintain glucose control outcomes, optionally providing TDM values as inputs may still improve the performance of the AMD systems. Moreover, TDM values may be commonly available for most users who first desire to utilize AMD systems, and may not represent a significant increase in complexity for the use of AMD systems.

Therefore, exemplary embodiments allow users to optionally provide this TDM as an input to the algorithms, allowing the system to incorporate modifications to both its initialization and subsequent behaviors, as needed. This can take a number of forms. The user may have previous data from (e.g.) manual medicament dosage data. Based on this, the user may have an estimate of their TDM values. Alternatively, a physician may estimate the TDM value for the user, and a physician-input TDM may be received by the system.

As a further alternative, the user's physician may develop a full basal profile, which may be input into the system. A basal profile, also known as a basal rate profile, is a term commonly used in the context of insulin therapy for people with diabetes. The basal profile refers to the programmed schedule of insulin delivery that provides a continuous, low level of insulin to the body to meet the basal metabolic needs of the individual throughout the day and night. The basal profile is an important aspect of medicament therapy for people with diabetes, as it helps to maintain consistent blood glucose levels and prevent high or low blood sugar episodes.

In an automated medicament delivery device 108, the basal profile may be set up by the healthcare provider or the patient, and it specifies the amount and timing of medicament delivery at different times of the day. The basal rate is typically adjusted based on individual needs and can vary depending on factors such as physical activity, stress, and mealtime insulin doses. Based on the amounts and timings of medicament delivery in the basal profile, the TDM may be calculated.

Depending on how a manual TDM value is entered, the component detection process 500 may apply weighting to determine a weighted TDM value at block 606. A user-input TDM might be less-trusted than the population-derived initial TDM value, since a user might make a mistake in calculating their TDM. A physician-generated or -input TDM may be more trusted than a user-input TDM, and indeed might be more trusted than the population-derived value since it has been customized to a particular user by a reliable source. A physician-generated basal profile might be the most trusted, to the point of (potentially) overriding the population-derived TDM.

Accordingly, If the user provides an optional TDM input that demonstrates a significantly increased insulin need compared to the low initial estimate, this can be reflected as follows. Note that the below equations are defined with respect to total daily insulin (TDI), assuming that insulin is the medicament. If another medicament is used, the appropriate TDM value for that medicament would be used in said equation. Hence, in the following, while the equations use "TDI", they also refer to "TDM".

The initial TDM estimate utilized by the AMD algorithm may apply a weighted sum of the system's default value, and the user's input TDM parameter. This can be captured as follows:

$$TDI_i = W \bullet TDI_d + (1 - W)TDI_m$$

Given that a user's input TDM ($TDM_{-M}$) will be manually entered, and may thus potentially be non ideal for the user, the weight for these parameters W may be heavily towards the default value $TDM_d$ (e.g., $W > 0.5$, preferably $0.6 < W < 0.95$, or more preferably $0.7 < W < 0.9$ ). A physician-input TDM might have a different weighting (e.g., $W < 0.5$, preferably $0.05 < W < 0.4$, or more preferably $0.1 < W < 0.3$). A TDM derived from an input basal profile might be weighed very heavily (e.g., $W < 0.3$, preferably $0 \leq W < 0.2$, or more preferably $0 \leq W < 0.1$)

Alternately or in addition, the system may be validated to be safe for a significant majority of users for two or more different sets of initial default parameters. If the user's input TDM value is below a certain threshold, the initial TDM may remain as the lowest initial parameter, whereas a higher input TDM by the user may allow the system to select a higher, but still safe, initial default parameter. This may be captured as follows:

| TDM category | Final $TDM_i$ | User input TDM |
|---|---|---|
| TDM(default, no input) | 10U | $TDM_m \leq 25U$ |
| TDM-(default, Low) | 20U | $25U < TDM_m \leq 40U$ |
| TDM(default, Medium) | 30U | $40U < TDM_m \leq 55U$ |
| TDM(Default, High) | 40U | $55U < TDM_m$ |

The values listed in each cell of this table are examples only, and the number of categories and each value within the cells can be customized.

According to some examples, the method further includes establishing an initial safe bolus limit based on the (weighted) TDM at block 608.

Specifically, the user's bolus deliveries may be limited to no more than an amount that may be potentially safe for the user. This may be calculated by assuming a risk of "no harm" in over-delivery as equivalent to an additional (e.g.) 0.5x basal delivery for up to 6 hours. This leads to a threshold of 3x basal over 6 hours, or, 0.75x hourly basal in a 90 minute period (peak time of insulin action). Therefore, an erroneous one-time over-delivery may be limited to no more than 0.75x the hourly basal for the lowest insulin need we are attempting to safely compensate for the user - such as 50% of the current estimated TDM that is safe for AMD systems. Therefore, the maximum bolus delivery at any one cycle may be set as:

$$b_{max}\left(i\right) = 0.75 \cdot \frac{0.5TDM_i(i)}{48} = 0.0078 \cdot TDM_i\left(i\right)$$

The delivery of the user's basal dose (which may be given in a series of background micro-doses over the course of a day) may then be constrained by $b_{max}$ such that the amount of basal dosage delivered in a given cycle (*i*) does not exceed $b_{max}$.

In this way, the initial estimate of the TDM may be calculated and an initial constraint limiting the amount of an acceptable bolus dose over a predetermined time window may be determined. Next, the medicament delivery system 100 may derive other constraints from the initial TDM estimate and may begin to deliver medicament based on the TDM and constraints.

An AMD algorithm also operates under a set of constraints to limit medicament delivery. However, these constraints, when initialized, may be modified to improve analyte outcomes without incurring additional risk to users.

FIG. 7A illustrates an example delivery process 700. Although the example delivery process 700 depicts a particular sequence of operations, the sequence may be altered without departing from the scope of the present disclosure. For example, some of the operations depicted may be performed in parallel or in a different sequence that does not materially affect the function of the delivery process 700. In other examples, different components of an example device or system that implements the delivery process 700 may perform functions at substantially the same time or in a specific sequence.

According to some examples, the method includes setting an initial medicament on board (MOB) constraint at block 702. The medicament-on-board constraint limits a maximum delivery of medicament to no greater than the amount required to bring the user to the target analyte concentrations.

However, the calculation for the amount needed for glucose reduction may be modified to account for the uncertain TDM. One example of an insulin-on-board constraint that accounts for uncertain TDM is shown below:

$$U_{max,IOB}(i) = \frac{CGM(i) - \max\left(120, target(i)\right)}{\frac{1600}{0.5 * TDI_{est}(i)}} - IOB(i)$$

Here, the heuristic rule of thumb for correction factor may be reduced by 50%, and the glucose target may be fixed at 120 mg/dL or higher to reduce risk of over-delivery. Other values may be used, i.e., a reduction value between 30% to 70%, or a glucose target between 100 mg/dL to 150 mg/dL.

A further constraint is the maximum cycle dose. According to some examples, the method includes setting initial maximum cycle dose at block 704.

At block 704, the total allowed automated insulin delivery constraints at any one cycle may also be modified to a fixed value or lower, with similar principles to the maximum basal delivery. Specifically, at any 3 hour cycle, the system may limit maximum total delivery basal to the threshold no greater than an overdose Low, or no more than an additional 1x basal over 6 hours, or 1x additional delivery per hour. Other values than the aforementioned, and those used in the following equation may be used. This may be translated to a fixed value based on a reduced estimate of the system's current TDM estimate, as follows:

$$U_{max,integral,3\ hours}(i) = 3 \cdot \frac{0.5TDI_{est}(i)}{48} = \frac{1.5TDI_{est}(i)}{48}$$

Yet another constraint is the upper bound, or maximum delivery, possible for the AMD system. According to some examples, the method includes adjusting the upper bound based on the user-input TDM at block 706.

In this scenario, the user's TDM inputs can be incorporated to modify the system's initial, conservative safety constraints. At a high level, these safety constraints may indicate a limitation to the upper bound, or maximum delivery by the AMD system. A modification of this initial conservative constraint may be affected as follows:

$$UB_i = \min\left(UB_{max},\ \left(UB_d + 0.25 \bullet \frac{TDI_m - TDI_d}{TDI_d}\right)\right)$$

Here, the upper bound may be increased by the proportion between the user's manually entered TDM versus the default TDM value, with the direct incorporation scaled by a tunable parameter (0.25 in this example).

Alternatively, as above, there may be one or more thresholds between the default conservative safety constraints versus a higher, but still conservative safety constraint demonstrated to be safe for the users, which may be selected if the user's TDM is higher than a threshold - similar to the table shown above in connection with the initialization process 600).

The above-described constraints may be adjusted over time as more data from the analyte sensor 110 becomes available. As the medicament delivery system 100 learns how the user responds to different doses of insulin, the constraints may be relaxed over time. In the absence of sufficient data, the constraints may be more limiting.

According to some examples, the method includes determining next dose amount at block 708. Using the MPC algorithm described above, the system attempts to select a dosage that minimizes a cost function. This generates an initial calculated value for the next dose. At decision block 710, this initial calculated value is compared to the above-described constraints (e.g., $U_{max,\ MOB}$, $U_{max,\ integral,\ 3\ hours}$, $UB_i$). If the initial calculated value does not exceed any of the constraints, then at block 714 the automated medicament delivery device 108 may generate and transmit a control signal configured to cause the medicament pump 122 to deliver an amount of medicament corresponding to the initial calculated value. If the initial calculated value exceeds one or more of the constraints, then the lowest constrained value is selected and at block 712 the automated medicament delivery device 108 may generate and transmit a control signal configured to cause the medicament pump 122 to deliver an amount of medicament corresponding to the constrained value.

Another key set of inputs typically utilized by AMD systems is the incorporation of announcement of disturbances to analyte (e.g., glucose) control that cannot be found by analyte concentration values - such as meal ingestion, exercise, and/or other factors. The detection of these events can be simplified through AMD system improvements on existing inputs, and/or automatic incorporation of other data sources to detect events without user input.

FIG. 7B illustrates an example event detection process 406. Although the example delivery process 700 depicts a particular sequence of operations, the sequence may be altered without departing from the scope of the present disclosure. For example, some of the operations depicted may be performed in parallel or in a different sequence that does not materially affect the function of the delivery process 700. In other examples, different components of an example device or system that implements the delivery process 700 may perform functions at substantially the same time or in a specific sequence.

According to some examples, the method includes retrieving an analyte trend at block 716. The analyte trend may be a series of analyte measurements as returned by the analyte sensor 110 over a given period of time (e.g., the past 30 minutes) or in certain contexts (e.g., following meal ingestion or exercise).

The AMD system may review different characteristics in analyte and medicament delivery trends to detect analyte control events. For instance, a rapid rise in glucose concentrations may be characteristic of a meal ingestion. A rapid reduction in glucose may be characteristic of an exercise. Models may be created based on typical responses of each user to a given medicament delivery value - and significant deviations from the trajectories calculated by the model can be utilized to detect a wide range of system behaviors.

According to some examples, the method includes evaluating recent analyte trends (e.g., over a predetermined recent time frame (also referred to as predetermined period of time), such as 5, 10, 15, 30, 60, ... minutes) at decision block 718. The trends may be compared to the aforementioned model to determined if the trend is indicative of (e.g.) meal ingestion or exercise. In some embodiments, the predetermine recent time frame is between 5 minutes to 120 minutes, more specifically 10 minutes to 60 minutes long.

For instance, if the trend indicates that the user's analyte levels have increased above a certain high threshold (or the current trend indicates that the high threshold will be exceeded within a predetermined period of time), this may indicate that the user is ingesting or has recently ingested a meal. Accordingly, at block 724 the system may flag a meal ingestion event. The detection of this event may have a number of consequences. The recent analyte trend may be used to further train the model to better identify meal ingestion events in the future. Alternatively or in addition, the medicament delivery system 100 may shift into a meal ingestion mode that causes an increased amount of medicament to be delivered, or may adjust system parameters or constraints to allow for additional medicament delivery over a predetermined horizon (e.g., based on the amount of time that a meal is predicted to result in increased analyte levels).

On the other hand, if the trend indicates that the user's analyte levels have decreased below a certain low threshold (or the current trend indicates that the low threshold will be met within a predetermined period of time), this may indicate that the user is exercising or has recently exercised. Accordingly, at block 728, the system may flag an exercise event. The detection of this event may have a number of consequences. The recent analyte trend may be used to further train the model to better identify exercise events in the future. Alternatively or in addition, the medicament delivery system 100 may shift into an exercise mode that causes a reduced amount of medicament to be delivered, or may adjust system parameters or constraints to allow for reduced medicament delivery over a predetermined horizon (e.g., based on the amount of time that the exercise is predicted to result in decreased analyte levels).

Furthermore, the AMD system may automatically incorporate a wide range of sensors without user input that are already present in the typical environment. This may allow the system to enter different states or adjust system parameters when an analyte trend has not yet been detected (or when a trend is not recognized by the models). For instance, a user's smartphone typically includes a wide range of sensors, such as accelerometry, GPS, and others. A user's smart watch may incorporate even wide range of health related sensors, such as heart rate, skin temperature, or others. An AMD system may incorporate each of these sensors into its environment to automatically detect events as they are available, dynamically improving its event detection without user inputs. For instance, if the accelerometer indicates a pattern typical of exercise, the AMD system may incorporate this data into its insulin deliveries. If the GPS system indicates that the user is within range of a restaurant, the system may increase its accuracy of detecting a meal disturbance.

Therefore, according to some examples, the method includes retrieving external sensor data at block 720. The external sensor data may include data from sensors on the control device 102 and/or other sensors, such as a smartwatch, fitness tracker, GPS, etc.

At decision block 722, the method may determine if the sensor data is indicative of the user eating or preparing to eat a meal. For example, if the GPS data indicates that the user is near a restaurant, the system may increase sensitivity to the detection of a meal ingestion event (e.g., relaxing parameters of the meal ingestion model so that the meal ingestion model is more likely to report a positive result). If historical data indicates that the user is present near a restaurant at which they have eaten before, or at which they have eaten frequently, the meal ingestion sensitivity may be further increased. Other examples of sensor data that might be indicative of a meal ingestion event may include the user entering meal parameters or checking a calorie count on a fitness device or app, the user turning on their kitchen lights at a time at which they have traditionally eaten a meal, etc. This may result in the detection of a meal ingestion event (block 724).

At decision block 726, the method may determine if the sensor data is indicative of the exercising or preparing to exercise. For example, if the GPS data indicates that the user is near a gym, park, pool, walking path, etc., the system may increase sensitivity to the detection of an exercise event (e.g., relaxing parameters of the exercise model so that the exercise model is more likely to report a positive result). If historical data indicates that the user is present near a gym, pool, park, or other location at which they have worked out before, or at which they have worked out frequently, the exercise sensitivity may be further increased. Other examples of sensor data that might be indicative of an exercise event may include the user accessing a workout program on a fitness device or app, an accelerometer or heart rate monitor indicating that the user has increased their activity level (especially at a time that the user has traditionally exercised), etc. This may result in the detection of an exercise event (block 728).

The AMD system may also adapt its non-glucose related system settings based on user interactions with the system. For instance, an AMD system, by necessity of ensuring user safety, will require certain alerts and/or alarms to be present for system failures. Depending on whether a user is prompt in responding to such alerts, the AMD system may reduce its frequency of alerts and alarms automatically to minimize disturbances to the user's daily lives without impacting the user's safety.

Alternately, if the user often does not interact with an available alert/alarm, the AMD system may increase its frequency of notifications to the users to ensure the user is made aware of potential risks to their diabetes care. For example, a user's blood glucose level might be low, but the user may be in a loud environment and cannot hear the alert. Alternatively, the user might be asleep. If the rate of interaction drops below a certain threshold, the system may increase the frequency of alerts or alarms.

FIG. 7C illustrates an example notification process 40. Although the example notification process 408 depicts a particular sequence of operations, the sequence may be altered without departing from the scope of the present disclosure. For example, some of the operations depicted may be performed in parallel or in a different sequence that does not materially affect the function of the notification process 408. In other examples, different components of an example device or system that implements the notification process 408 may perform functions at substantially the same time or in a specific sequence.

According to some examples, the method includes presenting alert at block 730. The alert may be indicative of, and sent in response to the detection of, an analyte control event (e.g., the user's blood glucose rising above or dropping below a predetermined threshold) or may be a system alert (e.g., indicating a problem with the medicament delivery system 100).

The system may measure how long it takes for the user to respond to the alert. Accordingly, in some examples, the method includes starting timer at block 732. Starting a timer may refer to actively starting a clock that counts up from zero or down from a predetermined number, or may simply involve noting the time at which the timer was started for later comparison to the current time.

The user may or may not respond to the alert by interacting with the medicament delivery system 100. At decision block 734, the system may determine if user input has been received.

If the user does not respond within a predetermined maximum period of time, or the user does respond but after a predetermined high response time threshold, then processing may proceed to block 736 and the medicament delivery system 100 may maintain or increase a parameter indicative of alert frequency. An increased alert frequency parameter may decrease the time until an alert is re-issued, or may cause the alert to be issued sooner (e.g., the threshold for detecting the analyte control event may be adjusted so that the user is alerted sooner, such as when their blood glucose level has approached but not yet reached the previous event threshold).

In some embodiments, the system may, a block 736, escalate the alerts or alarms. For example, the system may increase the volume of the alert, or add vibration to the alert. The system may alert additional devices (e.g., if the original alert was presented through a sound/vibration on the automated medicament delivery device 108, an escalated alert may include noise or vibration issued from the control device 102). In further embodiments, if a user does not respond to alerts and the analyte sensor 110 is transmitting signals consistent with a dangerous condition, the system may transmit an alert to the user's designated emergency contact or to a physician or medical care facility.

In further embodiments, a low rate of interactivity might also indicate that the user is not using their pump as normally. In response, the automated medicament delivery device 108 may taper down medicament delivery. For example, if the user is in a car accident or in a hospital, it may not be safe to deliver normal amounts of medicament to the user. In these circumstances, the automated medicament delivery device 108 may reduce and eventually eliminate (or reduce to a minimum safe basal value) the amount of medicament being delivered.

On the other hand, if the user does respond to the notification in a timely manner (less than a low threshold amount of time), then the alert frequency may be decreased at block 738.

The decision at decision block 734 may be made based on historical data. For example, the user's response times over a predetermined time horizon may be averaged, and the alert frequency may be adjusted based on the average. This may prevent the alert frequency from being changed if the user was unable to respond to an alert due to an isolated event (e.g., receiving a phone call) or if an otherwise slow responder happened to respond quickly to a single notification.

Once the TDM and constraints are initialized, they can be rapidly adapted based on data from the analyte sensor 110 to improve user outcomes. As an initial matter, this covers the AMD system's short-term adaptivity, where a conservative medicament delivery at the initiation of AMD system adapts within a short period (such as every 3 or 6 hours) to increase delivery for users with higher medicament needs based on analyte feedback. This change in the system may update the system's clinical parameters, such as basal medicament needs, analyte concentrations, or maximum medicament delivery limits, among others. The adaptivity period may be decreased, so that the algorithm adapts more quickly, under certain circumstances.

FIG. 8 illustrates an example short term adaptation process 800. Although the example short term adaptation process 800 depicts a particular sequence of operations, the sequence may be altered without departing from the scope of the present disclosure. For example, some of the operations depicted may be performed in parallel or in a different sequence that does not materially affect the function of the short term adaptation process 800. In other examples, different components of an example device or system that implements the short term adaptation process 800 may perform functions at substantially the same time or in a specific sequence.

According to some examples, the method includes starting timer at initialization at block 802. Starting a timer may refer to actively starting a clock that counts up from zero or down from a predetermined number, or may simply involve noting the time at which the timer was started for later comparison to the current time. The medicament delivery system 100 may execute adaptation logic when the timer reaches the end of an adaptation threshold. The timer may be set to adapt the parameters, for example, every 3, 4, 5, or 6 hours. This value may be accelerated as described below.

There is, however, a situation in which it might be useful to adapt immediately. If the user's analyte levels drop below or rise above predetermined danger thresholds (e.g., a blood glucose concentration below 40 mg/dL or above 400 mg/dL, respectively, indicated by a "YES" at decision block 804), then processing may proceed to block 814 and the medicament delivery system 100 may adapt immediately.

According to some examples, the method includes executing one or more medicament delivery cycles at block 806. The medicament delivery system 100 may execute one or more treatment cycles before re-evaluating whether to adapt (or may re-evaluate after predetermined periods of time, such as every 5, 10, 15, 30, etc. minutes).

According to some examples, the method includes reducing the adaptation threshold based on current and historical analyte at block 808. In an AMD system with no (or limited) inputs, the AMD system must initialize conservatively, leading to potentially safe delivery for users with low insulin needs, but sub-optimal behavior for users with high insulin needs. Instead, the rate of adaptivity for the AMD system may be modified based on the current and historical glucose. In one embodiment, this may be captured as the number of remaining hours until the system executes its next adaptivity routine:

$$H_{adapt}(i) = P_{adapt}\left(1 + N_{adapt}\right) - \frac{1}{12}\left(i + N_{CGM_{i-12P_{adapt}\ldots i}>180} + 2\left(N_{CGM_{i-12P_{adapt}\ldots i}\leq 55} + N_{CGM_{i-12P_{adapt}\ldots i}\geq 300}\right)\right)$$

Here, the period of adaptivity, in hours, may be decreased by 5 minutes every control cycle, and further reduced by the number of cycles of hyperglycemia and hypoglycemia - with the cycles of hyperglycemia above 300 mg/dL and hypoglycemia below 55 mg/dL resulting in accelerating the adaptivity rate by 3 times the standard rate. Other threshold values for hyperglycemia, i.e., 280 mg/dL, or hypoglycemia, i.e., 65 mg/dL and for accelerating the adaptivity rate (i.e., by 2 times to 4 times) may be used. These values are exemplary only, and both the thresholds and the acceleration rate may be adjusted as needed.

Alternately or in addition, the adaptation threshold can also be modified based on the number of times the user interacts with the system, as indicated by the number of user's boluses, in a similar manner:

$$H_{adapt}(i) = P_{adapt}\left(1 + N_{adapt}\right) - \frac{1}{12}\left(i + 3N_{bolus_{i-12P_{adapt}\ldots i}}\right)$$

Here, each user bolus since the previous adaptation may result in an acceleration of the adaptivity rate by 4 times the standard rate. This principle can be extended to the emergency threshold determination at decision block 804: if the user interacts with the system multiple times within a predetermined time period (e.g., requests 10 or more bolus doses within a relatively short period of time), the system may detect an emergency condition and immediately adapt.

According to some examples, the method further includes adjusting the adaptation threshold based on a difference between a manual and default TDM at block 810.

The initial TDM estimate utilized by the AMD algorithm may be adapted to the user's estimates once the system gains sufficient history of the user's insulin delivery. The duration of time before the system determines there is sufficient insulin history may be modified by the user's initial TDM inputs, if the system's ongoing insulin delivery history matches the user's TDM inputs. This may be captured (for example) as follows:

$$T_a\left(j\right) = T_{a,D} - 12 \bullet \max\left(0, \left(1 - \min\left(1, \frac{j}{288}\right) \bullet \frac{\left|TDI_m - \frac{\sum_{k=1}^{j} I(k)}{j} \bullet 288\right|}{TDI_m}\right)\right)$$

Here, the default time that the system may wait before the system begins adapting its TDM input, $T_{a,D}$ may be modified by the difference between the TDM manually entered by the user versus the actual insulin delivery history by the user, capped to at most (e.g.) 12 hours and scaling until the system has at least (e.g.) 1 day, or 288 cycles, of data. These values are exemplary only, and may be tuned as needed based on the application.

At decision block 812, the system determines if the adaptation threshold (potentially accelerated as noted above) has been reached. If so, processing proceeds to block 814 and the system initiates adaptation logic. If not, processing returns to block 804 and the system again determines whether the user's analyte levels have breached an emergency threshold (thus triggering immediate adaptation).

According to some examples, the method includes adapting parameters of the automated medicament delivery algorithm at block 814. The parameters may include, for example, basal medicament needs, analyte concentrations, medicament strength, maximum medicament delivery limits, or a total daily medicament value, among other possibilities.

The parameters may be adapted based on the analyte measurement history and trends. For example, if the analyte measurement history indicates that the user is not receiving a sufficient amount of medicament to keep analyte levels within a target range, the basal medicament dosage may be increased (and/or the maximum medicament delivery limits may be increased, and/or the total daily medicament value may be increased and/or the medicament strength may be increased). In a similar manner, if the user is receiving too much medicament, these values may be lowered.

Adaptation also covers the AMD system's long term adaptivity, as changes in the user's physiology, such as puberty, pregnancy, and/or illness, that result in persistent, long term changes required for the AMD system's parameters. FIG. 9 depicts the long-term adaptation process of FIG. 6 in more detail.

At block 902, the system may receive long-term dosing information and analyte sensor data that, together, describe how the user's response to their medicament regimen has changed over a predetermined period of time (e.g., 30 days, 40 days, 50 days, 60 days, 3 months, 6 months, one year, etc.). This long term change may be calculated using changes in the trends of the available data for the user, such as previous analyte and/or medicament delivery history.

In some cases, the user may also desire to modify the system's current behavior by entering a new TDM estimate at decision block 904, after the AMD system has started utilizing its own insulin delivery history for calculation of the user's insulin needs. If the user has not provided an updated TDM estimate, then processing may proceed to block 906 and the system may adapt system parameters (e.g., the same system parameters as may be adapted in the short term adaptation process 800) to address long term trends.

If the user did provide an updated TDM estimate at decision block 904, the user's input TDM parameter may also be incorporated as an additional parameter in the ongoing adaptation of the TDM value. In one embodiment, this may be considered as follows:

$$TDI_k = \left(1 - \frac{D_k}{3}\right)TDI_{k-1} + \frac{D_k}{3}TDI_{hist,k}$$

Here, $TDM_k$ indicates the actual TDM that would be utilized by the AMD system at the kth iteration of adaptation, and $D_k$ represents the duration of time of "new" history, in days, that is valid for the historical TDM used in this adaptation, or $TDM_{hist,k}$. The user's manual TDM input may be incorporated into this historical TDM, as follows:

$$TDI_{hist} = (1 - W_a)I_{tot,k} + W_a\frac{\sum_{q=1}^{N} TDI_{m,q}}{N}$$

Where the historical TDM is generated by weighted sum of the average daily total insulin needs in the kth cycle and an average of the user's input TDM values. The weighted sum may weigh more heavily towards the historical estimate the longer the system has had the user's insulin history, with potential values being calculated as follows:

$$W_a = \max\left(\min\left(0.3, 1 - \frac{D_k}{60}\right), 0.05\right)$$

Here, the maximum weight provided to the user's input TDM is 0.3, the minimum weight is 0.05, and the weight may scale linearly between approximately 42 days and 57 days of system use - with the minimum weight applied once more than 57 days of system use.

Each of the terms in the aforementioned equations may be adjusted as needed based on the application.

In the AMD algorithm described above in connection with FIG. 4 - FIG. 9, the user's current glucose concentrations and existing medicament-on-board (MOB) may be used to estimate the extent of acceptable additional medicament delivery. However, this method does not directly account for the eventual impact of remaining MOB on reduction in glucose concentrations, and does not incorporate the impact of meals on increases in glucose concentrations.

In the exemplary embodiment discussed below, the determination of whether the user has currently received sufficient insulin may be made by incorporating these two factors (impact of remaining MOB and impact of meals) directly into the user's glucose concentrations, to estimate the user's final glucose concentration if the impact of both carbohydrate ingestion and existing MOB are fully realized.

Exemplary logic for carrying out a technique that accounts for MOB and impact of meals on increases in glucose concentrations is depicted in FIG. 10A -FIG. 10D. The logic may be stored as instructions on a non-transitory computer-readable medium. The instructions may be configured to be performed on a processor of a computing device, such as the automated medicament delivery device 108 or the control device 102 of FIG. 1. Although the logic is depicted in terms of certain actions performed in a particular order, it will be understood that exemplary embodiments may employ more or fewer actions than depicted, in the same or a different order if the changes do not materially affect the function of the routine. Furthermore, the actions may be performed serially or in parallel, as appropriate, and may be split between multiple devices or performed on a single device.

An example will be provided alongside the description of the logic. The example is meant to be illustrative only; exemplary embodiments are not limited to the specific calculations performed in the example.

According to some examples, processing may begin at start block 1002. Start block 1002 may be performed, for example, upon system startup. In some embodiments, the system may wait until a certain amount of analyte measurement history and/or medicament delivery history has been obtained. Thus, processing may proceed after a predetermined period of time has elapsed since device startup (e.g., 90 minutes).

At start block 1002, the system may have certain initial data available to it. This data may include a known or assumed total daily medicament (TDM) value, a known or assumed previous carbohydrate ingestion history (or this may be calculated at block 1004), and a current glucose concentration which may be obtained from the analyte sensor 110 or an analyte test such as a finger-stick test. The TDM value may represent an amount of basal dose delivered to the user over a predetermined period of time (e.g., 24 hours) plus an amount of bolus doses delivered to the user over the predetermined time period. TDM may be obtained from previous device data (e.g., based on a history from the user's current automated medicament delivery device 108 or a previous automated medicament delivery device 108 used by the user, or data stored on the control device 102), may be determined from manual injection data (such as multiple daily injection, or MDI, data), or may be estimated by a medical professional, among other possibilities. The system may also have access to a target blood glucose level for the user. The system may further be able to access or determine the user's current medicament-on-board level,

For instance, assume that a device is initialized with a target blood glucose level of 110 mg/dL and a recent measured blood glucose level of 200 mg/dL. The system may have a record of 100 g of carbohydrate ingestion during a meal 3 hours ago. The user's TDM is initialized to 20 units/day, and the AMD system may estimate the user's current MOB at 2 units.

According to some examples, the method includes determining an impact of carbohydrate ingestion at block 1004. In some embodiments, this may be performed in a two-step process as shown in FIG. 10B. First, in block 1014, the user's ingestion of carbohydrates (e.g., in grams of carbohydrates) may be converted into current remaining carbohydrates-on-board, or COB, by estimating an absorption and/or decay rate of the ingested carbohydrates. In one embodiment, this decay rate may be estimated as a linear decay over X hours, such as 5 hours. In this instance, the current remaining COB may be estimated as follows:

$$COB(i) = \sum_{k=1}^{N} M_N \cdot \max\left(0, \left(1 - \frac{(i - j_{M_N})}{60}\right)\right)$$

Here, the remaining grams of carbohydrates for the $N^{th}$ ingested meal is calculated by multiplying the meal's original carbohydrate content $M_N$, by the remaining proportion of the 5 hours of duration of decay, which corresponds to 60 cycles at 12 5-minute cycles per hour, or the current 5-minute cycle *i* minus the cycle that the meal was ingested, of $j_{M,N}$. Other cycles length may be used, i.e, a cycle length between 1 min to 15 minutes.

The decay rate need not necessarily be a linear decay rate. It could be, for example, an exponential decay rate. The decay rate may also vary over time based on the circumstances, and may be represented by a model of carbohydrate decay.

Using the above example input data and the equation noted above, the user's current COB value may be estimated at:

$$100 \text{ grams} * \max(0, (1 - (36-0)/60) = 100 * (1 - 36/60) = 40 \text{ g}$$

In this example, we assume that the meal was eaten at time t = 0 (the 0th 5-minute cycle), which was 3 hours (or 36 5-minute cycles) ago. The same result would be obtained if, for example, the calculation were performed if the user ate at *t* = 1 hour (the 12th 5-minute cycle) and the calculation were done three hours later at time *t* = 4 hours (the 48th 5-minute cycle).

Next, in block 1016, the COB for previous meals can be converted into the glucose impact of the meals, as follows:

$$G_{meal}(i) = M_f COB(i)$$

where:

$$M_f = \frac{\left(\frac{500}{TDI}\right)^{-1} \cdot 1800}{TDI} = \frac{1800}{500} = 3.6$$

Note that the above equation is defined with respect to total daily insulin (TDI), assuming that insulin is the medicament. If another medicament is used, the appropriate TDM value for that medicament would be used. As mentioned before, the 1800 and 500 are rules commonly used, but may be varied.

Here, $M_f$ can be calculated in terms of mg/dL of glucose that will be increased per gram of remaining COB based on heuristic rules of thumb for IC ratios, such as the 500 rule, and their conversion into glucose absorption based on a correction factor, such as the 1800 rule. These heuristics are exemplary only, and will typically vary on a case-by-case basis. For example, the 1800 rule is a common rule which defines a person's insulin sensitivity by dividing 1800 by the total number of short-acting insulin units that they administer per day. Under this heuristic, a person who takes 30 units per day of insulin would have a sensitivity factor of 30 meaning that one unit of insulin would reduce glucose concentration by 60 mg/dL:

```
1800 / 30 = 60
```

Meanwhile, the 500 rule quantifies an estimated insulin-to-carbohydrate ratio, which estimates how many grams of carbohydrates a unit of insulin will address by dividing 500 by the user's TDM value. For example, a user taking 50 units of insulin would have an insulin-to-carbohydrate ratio of:

```
500 / 50 = 10
```

meaning that one unit of insulin would cover about 10 grams of carbohydrates.

Both the 1800 rule and the 500 rule are expedients that are general estimates applicable across a large population. However, these values may vary from individual-to-individual, and so the present invention is not limited to the specific values of 1800 and 500 for performing the above calculations.

Returning to the above example, the user's $G_{meal}(i)$ value would be estimated at:

```
3.6 * 40 = 144 mg/dL
```

In some cases, the system may not have access to a measure of the grams of ingested carbohydrates (e.g., because the user did not enter this value). In exemplary embodiments, an assumed amount of carbohydrates equivalent to the paired boluses that were delivered for those meals may instead be utilized. For instance, if 2% of the user's TDI is delivered as a bolus for a meal of unknown carbohydrate content, this can be converted into an assumed meal carbohydrate content as follows:

$$M_{N,assumed} = 0.02 \cdot TDI \cdot \frac{500}{TDI} = 10$$

Specifically, the raw # (number) of units of bolus can be entered as the coefficient of the correction factor equation (500/TDI) in the equation above, for any units of manual bolus delivered, as follows:

$$M_{N,assumed} = I_{b,meal} \cdot \frac{500}{TDI}$$

In this equation, the meal proportion of such a bolus $I_{b,meal}$ can be calculated as the user's manual bolus delivery request minus the correction amount needed at the time:

$$I_{b,meal} = \max\left(0, I_{b,manual}(i) - \left(\frac{CGM(i) - Target(i)}{1800/TDI}\right)\right)$$

Again, these equations utilize insulin as a medicament and hence incorporate a TDI value and a glucose reading from a continuous glucose monitor (CGM). Other medicaments would use their own suitable TDM values and/or analyte sensor values. Additionally, the 1800 rule and the 500 rule are used here, which may vary from user-to-user, and thus different values may be used.

According to some examples, the method next includes determining impact of remaining medicament on board at block 1006. The impact of remaining MOB can be calculated by simply multiplying the user's current MOB, which may be determined by the control device 102 or automated medicament delivery device 108 as described above, to the user's correction factor, as follows:

$$G_{IOB}(i) = IOB(i) \cdot \frac{1800}{TDI}$$

As before, this example uses insulin as the medicament and the 1800 rule. Other medicaments and sensitivity heuristics may be used as appropriate.

Following along with the previous example, the user's medicament on board impact $G_{IOB}(i)$ would be:

$$2 \times \frac{1800}{20} = 180\, mg/dL$$

This means that the net effect of the current COB and MOB, which represents the expected current glucose after all effects of consumed carbohydrates and medicament are accounted for, is:

200 (Current Glucose Level) + 144 ($G_{meal}$) - 180 ($G_{IOB}(i)$) = 164 mg/dL

According to some examples, the method next includes determining residual medicament needs at block 1008. This may be performed in a two-step process as shown in FIG. 10C.

First, at block 1018, the user's current total glucose deviation versus the target glucose can be calculated. One technique for calculating the current glucose deviation is as follows:

$$G_{\Delta}(i) = G(i) - Target(i) + G_{meal}(i) - G_{IOB}(i)$$

where *G(i)* is the user's glucose level at time *i*, *Target(i)* is the user's glucose target at time *i*, and $G_{meal}$ and $G_{IOB}$ are calculated as described above. Continuing our previous example, the user's $G_{\Delta}$ would be:

200 - 110 + 144 - 180 = +54 mg/dL above target

At block 1020, $G_{\Delta}$ can then be converted into the user's residual insulin needs, as follows:

$$I_{req}(i) = G_{\Delta}(i)\left(\frac{1800}{TDI}\right)^{-1}$$

These equations again use the 1800 rule heuristic and insulin as a medicament. Other medicaments and heuristics may be applied as appropriate. Continuing the previous example, the user's $I_{req}$ value would be:

$$54 \times \frac{1800}{20}^{-1} = 0.6\, U$$

According to some examples, the method next includes converting residual medicament needs into a change in the input TDM for AMD algorithm at block 1010. This may be performed in a two-step process as shown in FIG. 10D.

Given that this residual need specifies the user's additional medicament needs in the current moment, the user should have received this additional insulin need and/or reduced medicament delivery within the window of typical insulin action peak times, or for example 90 minutes. Therefore, at block 1022 the increased basal medicament delivery matching this insulin requirement may be calculated as follows:

$$b_\Delta(i) = \frac{I_{req}(i)}{1.5}$$

where 1.5 represents the exemplary 1.5 hours or 90 minutes.

Continuing with the above example, the user's increased basal delivery value would be:

$$\frac{0.6}{1.5} = 0.4\ units\ /\ hour$$

At block 1024, this additional basal need can be converted into a change in the user's TDM that may be utilized as an input to AMD algorithms, given the assumption that 50% of the user's TDM should be covered by basal delivery:

$$TDI_\Delta(i) = b_\Delta(i) \cdot 48$$

This equation uses insulin as a medicament and a 50% basal / 50% bolus split, such that the actual change in total daily insulin is equivalent to the estimated change in hourly basal converted into 24 hours, then further multiplied by 2 to account for the same proportion of boluses that will also contribute to the TDI. Other medicaments and splits may be used as appropriate to the user, and the durations may also be either converted to a full duration, or converted only to a fixed amount equivalent to the initial calculated duration, such as 1.5 hours.

Continuing the above example, the user's change in TDM would be:

$$0.4 \times 48 = 19.2\,U$$

According to some examples, the method includes incorporating the change in TDM into the AMD system's adaptivity schemes at block 1012.

The above $TDI_\Delta$ value may be a real-time estimate of the change in the user's TDI that may be utilized by the AMD system through a variety of factors, as discussed above in connection with FIG. 4 - FIG. 9. In one embodiment, this parameter may be calculated in real time once sufficient history is available (such as 90 minutes), and can be directly incorporated as a modification to the system's TDI estimate per predetermined increments (e.g., 1 minute, 5 minutes, 10 minutes, 60 minutes, etc.), such as:

$$TDI_{new}(i) = (1 - X) \cdot TDI(i) + X \cdot \big(TDI(i-1) + TDI_\Delta(i-1)\big)$$

where X is a tunable parameter, such as 0.005. This equation uses insulin as the medicament; other medicaments may be substituted and the appropriate TDM value used.

Continuing the above example, the new TDM value for the user would be:

```
(1-0.005)*20 + 0.005*(20 + 19.2) = 20.096U
```

Thus, the user's TDM value may be changed, in the next five-minute interval (or another predetermined interval, as appropriate to the AMD device), from 20 U to 20.096U.

In another embodiment, the $TDM_{new}$ parameter may be continuously calculated, but only utilized as a *new* estimate if the confidence interval of the $TDI_\Delta$ may be stabilized (e.g., the change in the $TDI_\Delta$ can remain within one standard deviation (other measures than one standard deviation are possible as threshold for determining stability)). This may be represented as:

$$TDI_{new}(i) = \begin{cases} TDI(i) + TDI_{\Delta}(i-1) & TDI_{\Delta}(i-1) - TDI_{\Delta}(i-2) \leq std\left(TDI_{\Delta}(min, \ldots i)\right) \\ TDI(i-1) & otherwise \end{cases}$$

Once $TDM_{new}$ is calculated, the algorithm may generate a control signal configured to update the TDM value in the memory of the control device 102 and/or automated medicament delivery device 108. The control signal may be transmitted to the control device 102 and/or automated medicament delivery device 108, e.g. through wired or wireless communication, over a signal bus, etc. The control signal may be configured to cause the AMD system to deliver medicament at a rate defined by the $TDM_{new}$ value. The control signal may be configured to cause the AMD system to calculate a medicament dose based on the $TDM_{new}$ value or to calculate a medicament rate defined by the $TDM_{new}$ value. For example, the control signal may be configured to cause a drive mechanism (such as a lead screw connected to a plunger or other suitable mechanism) of the medicament pump 122 to be driven by an amount commensurate with and based on the $TDM_{new}$ value.

The components and features of the devices described above may be implemented using any combination of discrete circuitry, application specific integrated circuits (ASICs), logic gates and/or single chip architectures. Further, the features of the devices may be implemented using microcontrollers, programmable logic arrays and/or microprocessors or any combination of the foregoing where suitably appropriate. It is noted that hardware, firmware and/or software elements may be collectively or individually referred to herein as "logic" or "circuit."

It will be appreciated that the exemplary devices shown in the block diagrams described above may represent one functionally descriptive example of many potential implementations. Accordingly, division, omission or inclusion of block functions depicted in the accompanying figures does not infer that the hardware components, circuits, software and/or elements for implementing these functions would necessarily be divided, omitted, or included in embodiments.

At least one computer-readable storage medium may include instructions that, when executed, cause a system to perform any of the computer-implemented methods described herein.

Some embodiments may be described using the expression "one embodiment" or "an embodiment" along with their derivatives. These terms mean that a particular feature, structure, or characteristic described in connection with the embodiment is included in at least one embodiment. The appearances of the phrase "in one embodiment" in various places in the specification are not necessarily all referring to the same embodiment. Moreover, unless otherwise noted the features described above are recognized to be usable together in any combination. Thus, any features discussed separately may be employed in combination with each other unless it is noted that the features are incompatible with each other.

With general reference to notations and nomenclature used herein, the detailed descriptions herein may be presented in terms of program procedures executed on a computer or network of computers. These procedural descriptions and representations are used by those skilled in the art to most effectively convey the substance of their work to others skilled in the art.

A procedure is here, and generally, conceived to be a self-consistent sequence of operations leading to a desired result. These operations are those requiring physical manipulations of physical quantities. Usually, though not necessarily, these quantities take the form of electrical, magnetic or optical signals capable of being stored, transferred, combined, compared, and otherwise manipulated. It proves convenient at times, principally for reasons of common usage, to refer to these signals as bits, values, elements, symbols, characters, terms, numbers, or the like. It should be noted, however, that all of these and similar terms are to be associated with the appropriate physical quantities and are merely convenient labels applied to those quantities.

Further, the manipulations performed are often referred to in terms, such as adding or comparing, which are commonly associated with mental operations performed by a human operator. No such capability of a human operator is necessary, or desirable in most cases, in any of the operations described herein, which form part of one or more embodiments. Rather, the operations are machine operations. Useful machines for performing operations of various embodiments include general purpose digital computers or similar devices.

Some embodiments may be described using the expression "coupled" and "connected" along with their derivatives. These terms are not necessarily intended as synonyms for each other. For example, some embodiments may be described using the terms "connected" and/or "coupled" to indicate that two or more elements are in direct physical or electrical contact with each other. The term "coupled," however, may also mean that two or more elements are not in direct contact with each other, but yet still co-operate or interact with each other.

Various embodiments also relate to apparatus or systems for performing these operations. This apparatus may be specially constructed for the required purpose or it may comprise a general purpose computer as selectively activated or reconfigured by a computer program stored in the computer. The procedures presented herein are not inherently related to a particular computer or other apparatus. Various general purpose machines may be used with programs written in accordance with the teachings herein, or it may prove convenient to construct more specialized apparatus to perform the required method steps. The required structure for a variety of these machines will appear from the description given.

It is emphasized that the Abstract of the Disclosure is provided to allow a reader to quickly ascertain the nature of the technical disclosure. It is submitted with the understanding that it will not be used to interpret or limit the scope or meaning of the claims. In addition, in the foregoing Detailed Description, it can be seen that various features are grouped together in a single embodiment for the purpose of streamlining the disclosure. This method of disclosure is not to be interpreted as reflecting an intention that the claimed embodiments require more features than are expressly recited in each claim. Rather, as the following claims reflect, inventive subject matter lies in less than all features of a single disclosed embodiment. Thus the following claims are hereby incorporated into the Detailed Description, with each claim standing on its own as a separate embodiment. In the appended claims, the terms "including" and "in which" are used as the plain-English equivalents of the respective terms "comprising" and "wherein," respectively. Moreover, the terms "first," "second," "third," and so forth, are used merely as labels, and are not intended to impose numerical requirements on their objects.

What has been described above includes examples of the disclosed architecture. It is, of course, not possible to describe every conceivable combination of components and/or methodologies, but one of ordinary skill in the art may recognize that many further combinations and permutations are possible. Accordingly, the novel architecture is intended to embrace all such alterations, modifications and variations that fall within the spirit and scope of the appended claims.

The components and features of the devices described above may be implemented using any combination of discrete circuitry, application specific integrated circuits (ASICs), logic gates and/or single chip architectures. Further, the features of the devices may be implemented using microcontrollers, programmable logic arrays and/or microprocessors or any combination of the foregoing where suitably appropriate. It is noted that hardware, firmware and/or software elements may be collectively or individually referred to herein as "logic" or "circuit."

It will be appreciated that the exemplary devices shown in the block diagrams described above may represent one functionally descriptive example of many potential implementations. Accordingly, division, omission or inclusion of block functions depicted in the accompanying figures does not infer that the hardware components, circuits, software and/or elements for implementing these functions would be necessarily be divided, omitted, or included in embodiments.

At least one computer-readable storage medium may include instructions that, when executed, cause a system to perform any of the computer-implemented methods described herein.

Some embodiments may be described using the expression "one embodiment" or "an embodiment" along with their derivatives. These terms mean that a particular feature, structure, or characteristic described in connection with the embodiment is included in at least one embodiment. The appearances of the phrase "in one embodiment" in various places in the specification are not necessarily all referring to the same embodiment. Moreover, unless otherwise noted the features described above are recognized to be usable together in any combination. Thus, any features discussed separately may be employed in combination with each other unless it is noted that the features are incompatible with each other.

With general reference to notations and nomenclature used herein, the detailed descriptions herein may be presented in terms of program procedures executed on a computer or network of computers. These procedural descriptions and representations are used by those skilled in the art to most effectively convey the substance of their work to others skilled in the art.

A procedure is here, and generally, conceived to be a self-consistent sequence of operations leading to a desired result. These operations are those requiring physical manipulations of physical quantities. Usually, though not necessarily, these quantities take the form of electrical, magnetic or optical signals capable of being stored, transferred, combined, compared, and otherwise manipulated. It proves convenient at times, principally for reasons of common usage, to refer to these signals as bits, values, elements, symbols, characters, terms, numbers, or the like. It should be noted, however, that all of these and similar terms are to be associated with the appropriate physical quantities and are merely convenient labels applied to those quantities.

Further, the manipulations performed are often referred to in terms, such as adding or comparing, which are commonly associated with mental operations performed by a human operator. No such capability of a human operator is necessary, or desirable in most cases, in any of the operations described herein, which form part of one or more embodiments. Rather, the operations are machine operations. Useful machines for performing operations of various embodiments include general purpose digital computers or similar devices.

Some embodiments may be described using the expression "coupled" and "connected" along with their derivatives. These terms are not necessarily intended as synonyms for each other. For example, some embodiments may be described using the terms "connected" and/or "coupled" to indicate that two or more elements are in direct physical or electrical contact with each other. The term "coupled," however, may also mean that two or more elements are not in direct contact with each other, but yet still co-operate or interact with each other.

Various embodiments also relate to apparatus or systems for performing these operations. This apparatus may be specially constructed for the required purpose or it may comprise a general purpose computer as selectively activated or reconfigured by a computer program stored in the computer. The procedures presented herein are not inherently related to a particular computer or other apparatus. Various general purpose machines may be used with programs written in accordance with the teachings herein, or it may prove convenient to construct more specialized apparatus to perform the required method steps. The required structure for a variety of these machines will appear from the description given.

It is emphasized that the Abstract of the Disclosure is provided to allow a reader to quickly ascertain the nature of the technical disclosure. It is submitted with the understanding that it will not be used to interpret or limit the scope or meaning of the claims. In addition, in the foregoing Detailed Description, it can be seen that various features are grouped together in a single embodiment for the purpose of streamlining the disclosure. This method of disclosure is not to be interpreted as reflecting an intention that the claimed embodiments require more features than are expressly recited in each claim. Rather, as the following claims reflect, inventive subject matter lies in less than all features of a single disclosed embodiment. Thus the following claims are hereby incorporated into the Detailed Description, with each claim standing on its own as a separate embodiment. In the appended claims, the terms "including" and "in which" are used as the plain-English equivalents of the respective terms "comprising" and "wherein," respectively. Moreover, the terms "first," "second," "third," and so forth, are used merely as labels, and are not intended to impose numerical requirements on their objects.

What has been described above includes examples of the disclosed architecture. It is, of course, not possible to describe every conceivable combination of components and/or methodologies, but one of ordinary skill in the art may recognize that many further combinations and permutations are possible. Accordingly, the novel architecture is intended to embrace all such alterations, modifications and variations that fall within the spirit and scope of the appended claims.

Although the present invention is defined in the attached claims, it should be understood that the present invention can also (alternatively) be defined in accordance with the following aspects:

1. A computer-implemented method comprising:

   receiving a total daily medicament value representing an amount of medicament currently being delivered to a user by an automated medicament delivery device on a daily basis;
   determining the user's ingestion of carbohydrates over a predetermined period of time;
   determining the user's current remaining medicament-on-board;
   using the ingestion of carbohydrates and the current remaining medicament-on-board to update the total daily medicament value; and
   causing the automated medicament delivery device to deliver medicament to the user based on the updated total daily medicament value.

2. The computer-implemented method of aspect 1, further comprising calculating an amount of carbohydrates-on-board for the user based on the user's ingestion of carbohydrates in the predetermined period of time, and applying the carbohydrates-on-board when updating the total daily medicament value.

3. The computer-implemented method of aspect 1, wherein determining the user's ingestion of carbohydrates over the predetermined period of time comprises receiving input from the user comprising an estimate of the amount of carbohydrates consumed in the predetermined period of time.

4. The computer-implemented method of aspect 1, wherein determining the user's ingestion of carbohydrates comprises estimating an amount of carbohydrates based on bolus medicament amounts delivered to the user during a meal.

5. The computer-implemented method of aspect 1, wherein using the current remaining medicament-on-board to update the total daily medicament value comprises determining an impact of the current remaining medicament-on-board by adjusting the current remaining medicament-on-board based on a medicament sensitivity factor.

6. The computer-implemented method of aspect 1, wherein using the ingestion of carbohydrates and the current remaining medicament-on-board to update the total daily medicament value comprises determining a residual medicament need for the user based on the user's expected current analyte level after an effect of the ingested carbohydrates and medicament-on-board is accounted for.

7. The computer-implemented method of aspect 6, wherein the residual medicament need is determined by adjusting the expected current analyte level based on a sensitivity factor.

8. The computer-implemented method of aspect 1, further comprising computing the total daily medicament value at predetermined intervals.

9. The computer-implemented method of aspect 8, wherein the total daily medicament value is updated only when a confidence interval of a change in the total daily medicament value is determined to be stable.

10. A non-transitory computer-readable medium storing instructions that, when executed by one or more processors, cause the processors to perform the method of any one of aspects 1 to 9.

11. An automated medicament delivery system comprising:
one or more processors; and
a non-transitory computer-readable medium storing instructions that, when executed the one or more processors, cause the processors to perform the method of any one of aspects 1 to 9.

## Claims

**1.** A non-transitory computer-readable medium storing instructions that, when executed by one or more processors, cause the processors to perform a method, the method comprising:

receiving a total daily medicament value representing an amount of medicament currently being delivered to a user by an automated medicament delivery device on a daily basis;
determining the user's ingestion of carbohydrates over a predetermined period of time;
determining the user's current remaining medicament-on-board;
using the ingestion of carbohydrates and the current remaining medicament-on-board to update the total daily medicament value; and
causing the automated medicament delivery device to deliver medicament to the user based on the updated total daily medicament value.

**2.** The system of claim 1, further comprising calculating an amount of carbohydrates-on-board for the user based on the user's ingestion of carbohydrates in the predetermined period of time, and applying the carbohydrates-on-board when updating the total daily medicament value.

**3.** The system of claim 1 or 2, wherein determining the user's ingestion of carbohydrates over the predetermined period of time comprises receiving input from the user comprising an estimate of the amount of carbohydrates consumed in the predetermined period of time.

**4.** The system of any of claims 1-3, wherein determining the user's ingestion of carbohydrates comprises estimating an amount of carbohydrates based on bolus medicament amounts delivered to the user to compensate a meal.

**5.** The system of any of claims 1-4, wherein using the current remaining medicament-on-board to update the total daily medicament value comprises determining an impact of the current remaining medicament-on-board on reduction in glucose concentrations by adjusting the current remaining medicament-on-board based on a medicament sensitivity factor.

**6.** The system of any of claims 1-5, wherein using the ingestion of carbohydrates and the current remaining medicament-on-board to update the total daily medicament value comprises determining a residual medicament need for the user based on the user's expected current analyte level after an effect of the ingested carbohydrates and medicament-on-board is accounted for.

**7.** The system of claim 6, wherein the residual medicament need is determined by adjusting the expected current analyte level based on a sensitivity factor.

**8.** The system of any of claims 1-7, further comprising computing the total daily medicament value at predetermined intervals.

**9.** The system of claim 8, wherein the total daily medicament value is updated only when a confidence interval of a change in the total daily medicament value is determined to be stable.

**10.** An automated medicament delivery system comprising:

one or more processors; and
the non-transitory computer-readable medium storing instructions of any of claims 1 to 9.

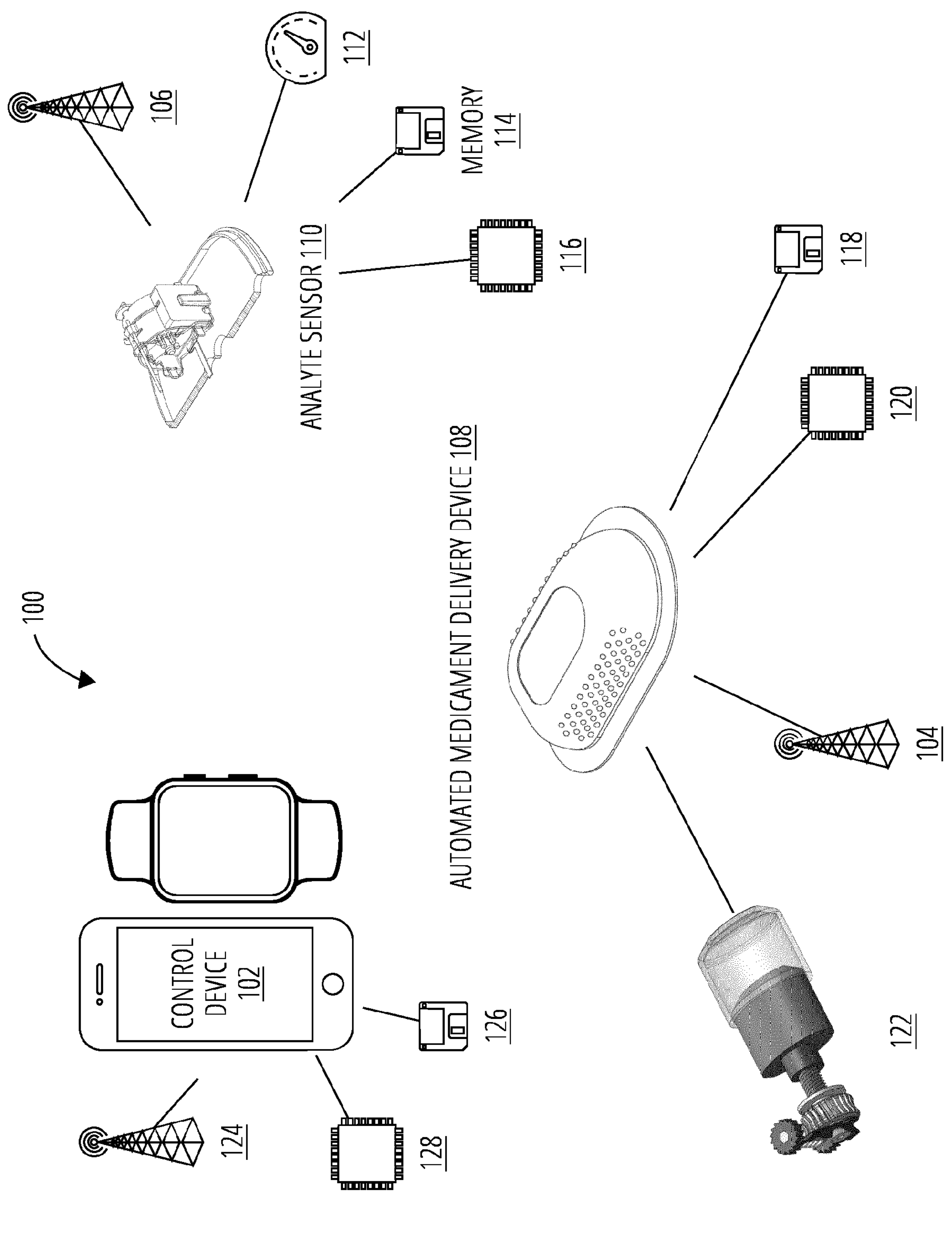
100
106
112
MEMORY
114
ANALYTE SENSOR 110
116
118
120
AUTOMATED MEDICAMENT DELIVERY DEVICE 108
104
CONTROL
DEVICE
102
126
124
128
122

FIG. 1

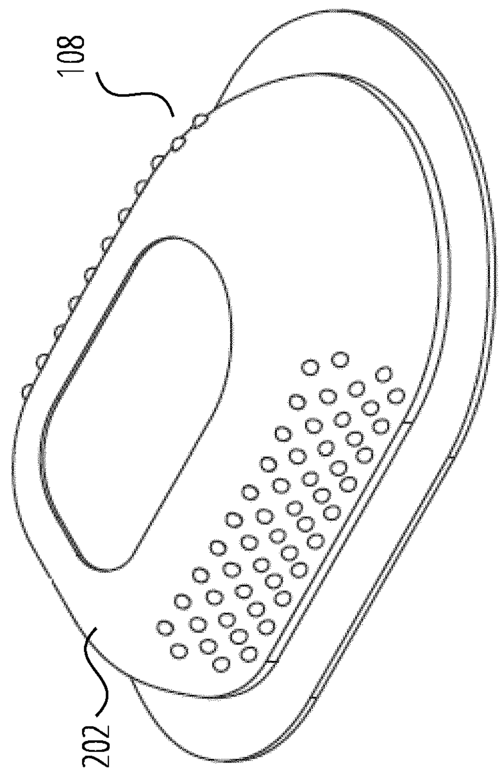


FIG. 2A

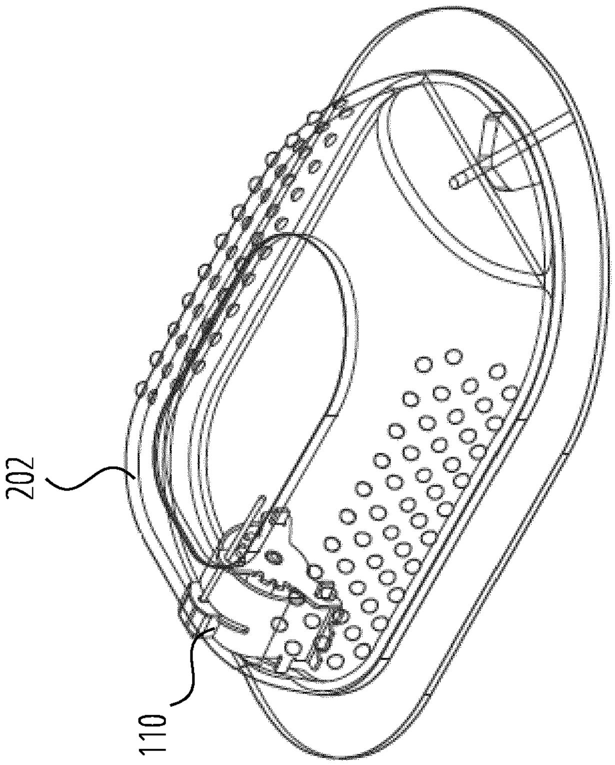


FIG. 2B

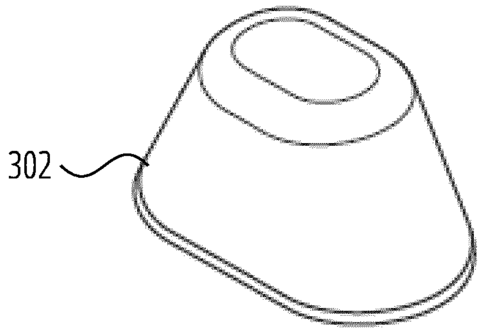


FIG. 3A

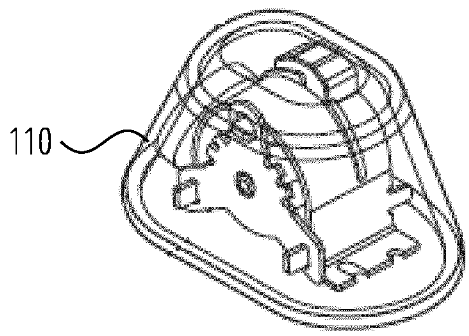


FIG. 3B

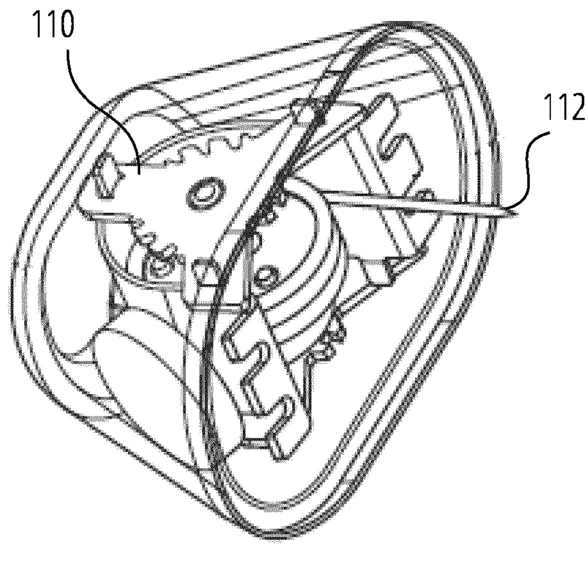


FIG. 3C

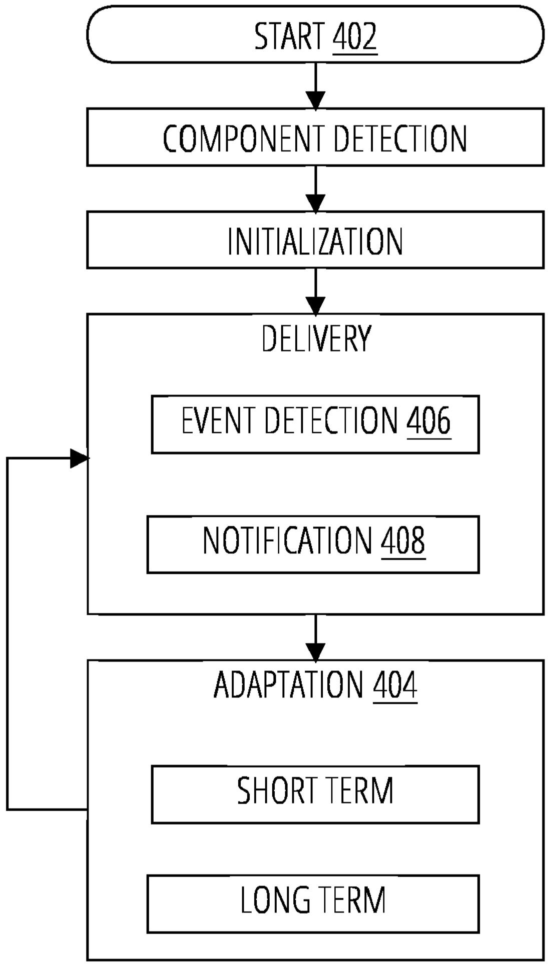
START 402
COMPONENT DETECTION
INITIALIZATION
DELIVERY
EVENT DETECTION 406
NOTIFICATION 408
ADAPTATION 404
SHORT TERM
LONG TERM

FIG. 4

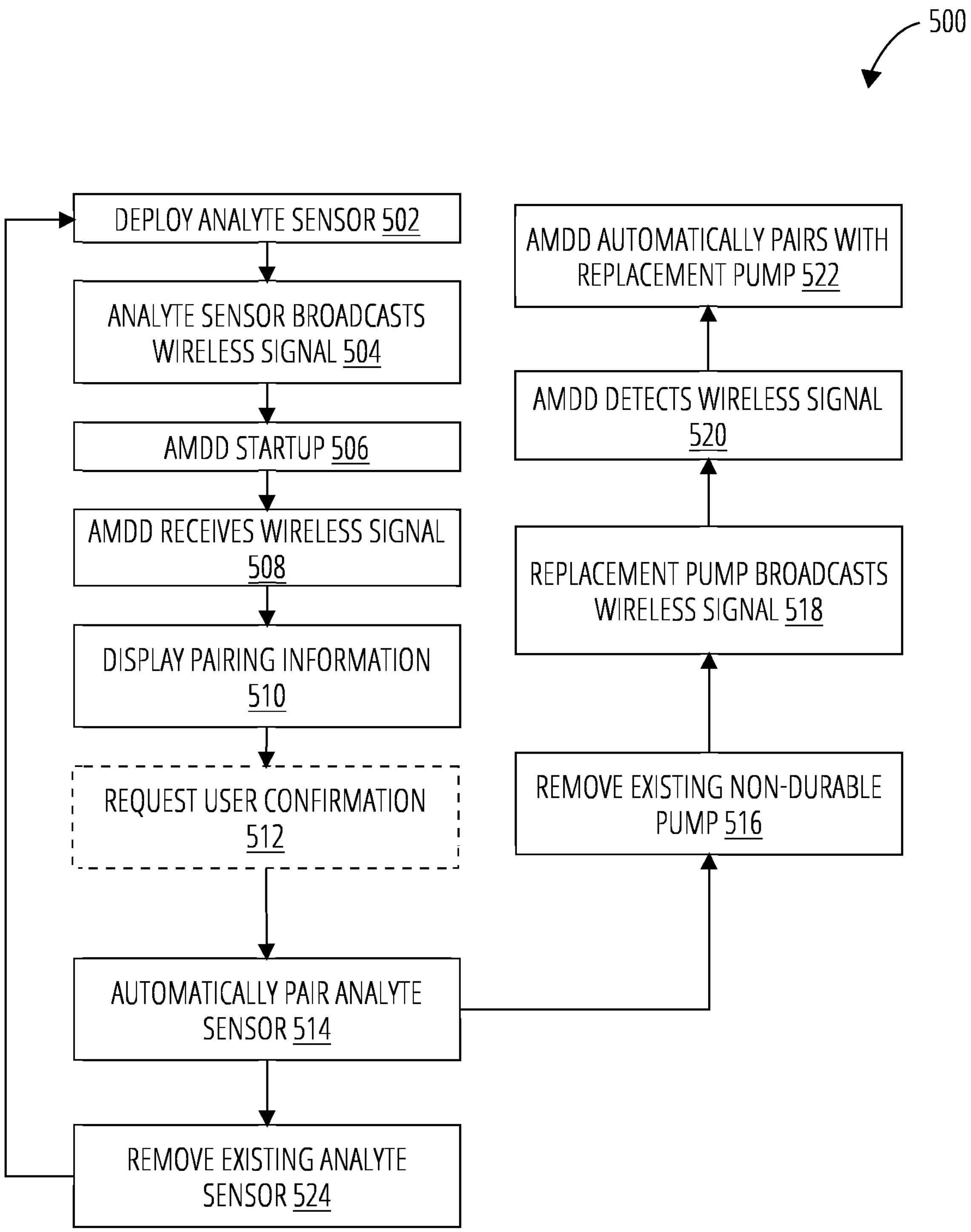
500
DEPLOY ANALYTE SENSOR 502
ANALYTE SENSOR BROADCASTS WIRELESS SIGNAL 504
AMDD STARTUP 506
AMDD RECEIVES WIRELESS SIGNAL 508
DISPLAY PAIRING INFORMATION 510
REQUEST USER CONFIRMATION 512
AUTOMATICALLY PAIR ANALYTE SENSOR 514
REMOVE EXISTING ANALYTE SENSOR 524
AMDD AUTOMATICALLY PAIRS WITH REPLACEMENT PUMP 522
AMDD DETECTS WIRELESS SIGNAL 520
REPLACEMENT PUMP BROADCASTS WIRELESS SIGNAL 518
REMOVE EXISTING NON-DURABLE PUMP 516

FIG. 5

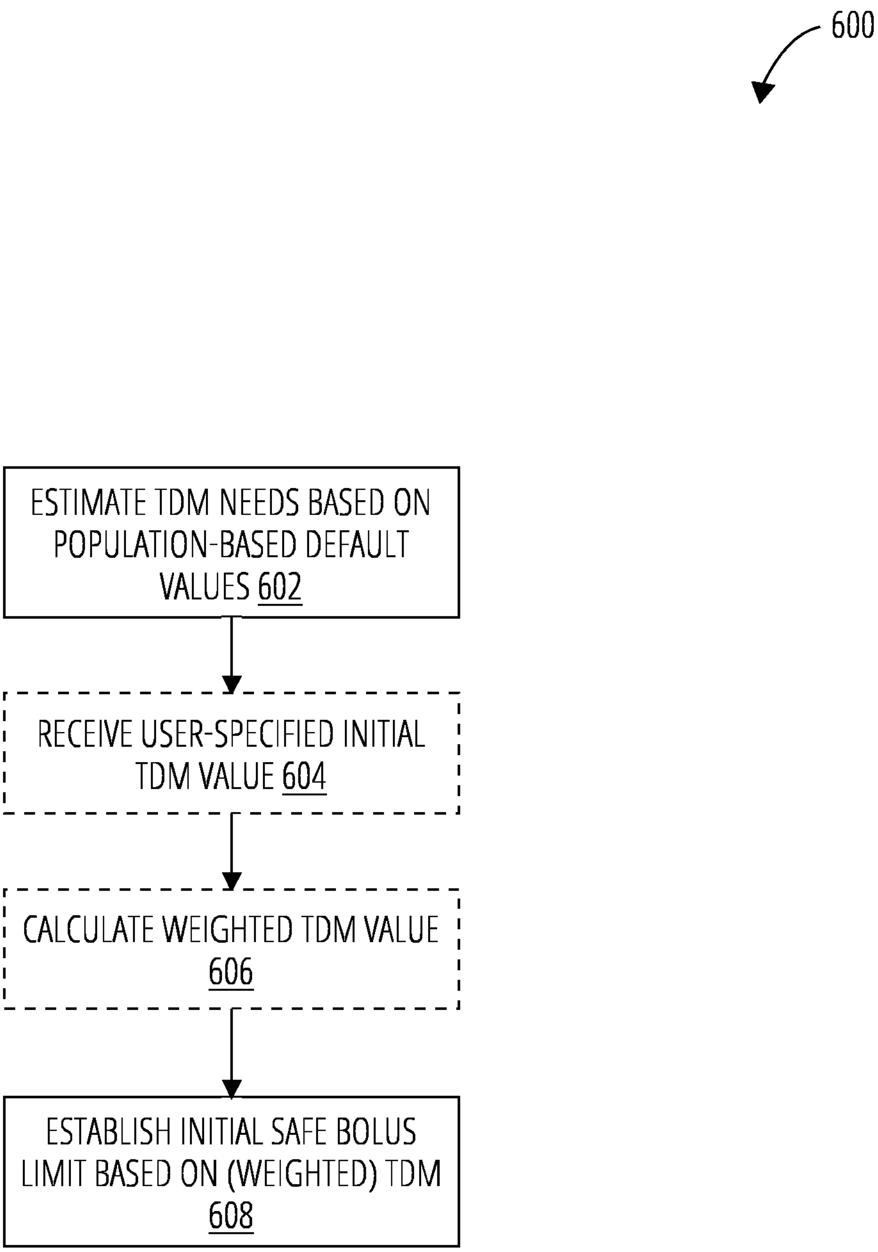
600
ESTIMATE TDM NEEDS BASED ON POPULATION-BASED DEFAULT VALUES 602
RECEIVE USER-SPECIFIED INITIAL TDM VALUE 604
CALCULATE WEIGHTED TDM VALUE 606
ESTABLISH INITIAL SAFE BOLUS LIMIT BASED ON (WEIGHTED) TDM 608

FIG. 6

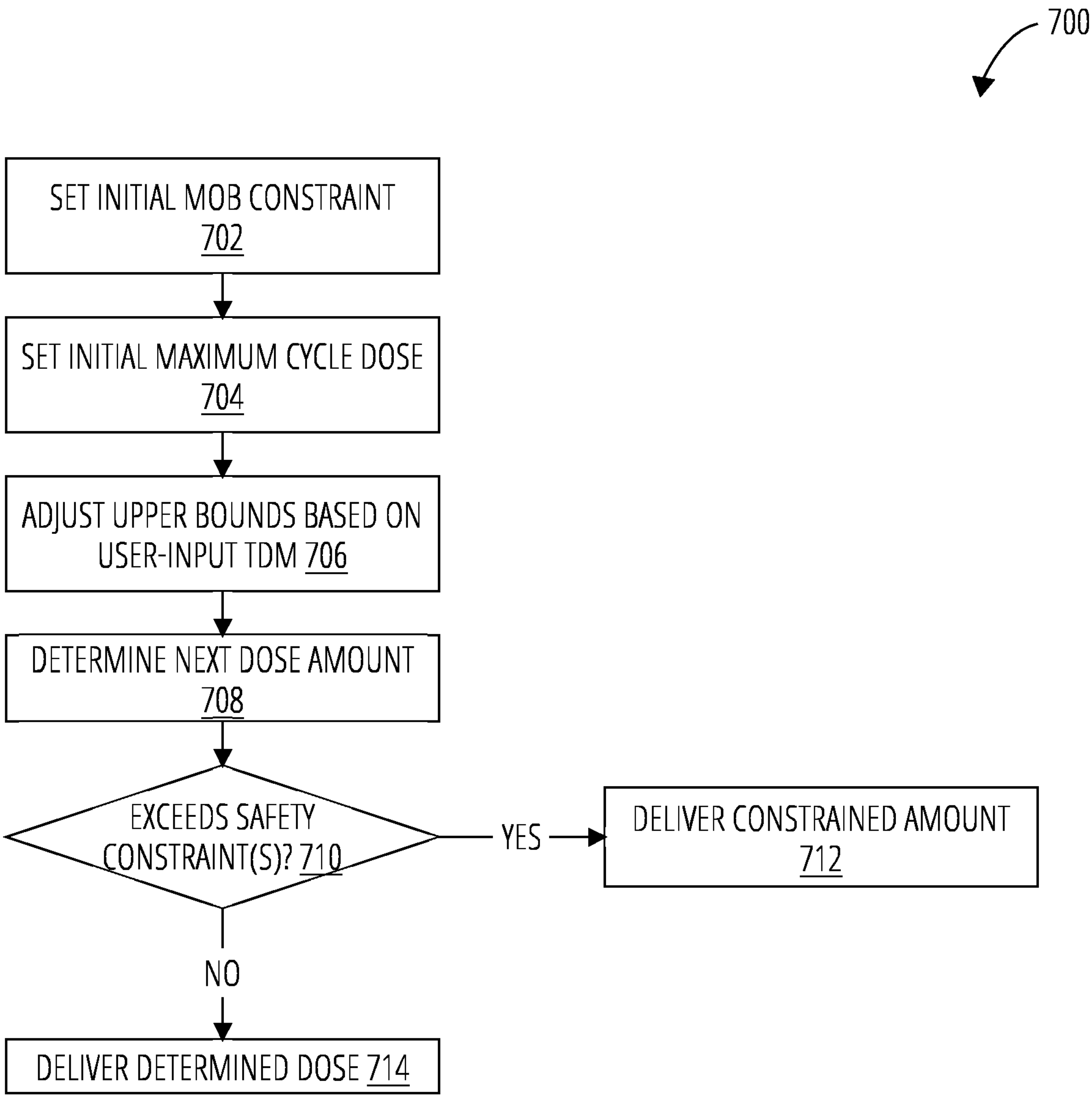
700
SET INITIAL MOB CONSTRAINT 702
SET INITIAL MAXIMUM CYCLE DOSE 704
ADJUST UPPER BOUNDS BASED ON USER-INPUT TDM 706
DETERMINE NEXT DOSE AMOUNT 708
EXCEEDS SAFETY CONSTRAINT(S)? 710
YES
DELIVER CONSTRAINED AMOUNT 712
NO
DELIVER DETERMINED DOSE 714

FIG. 7A

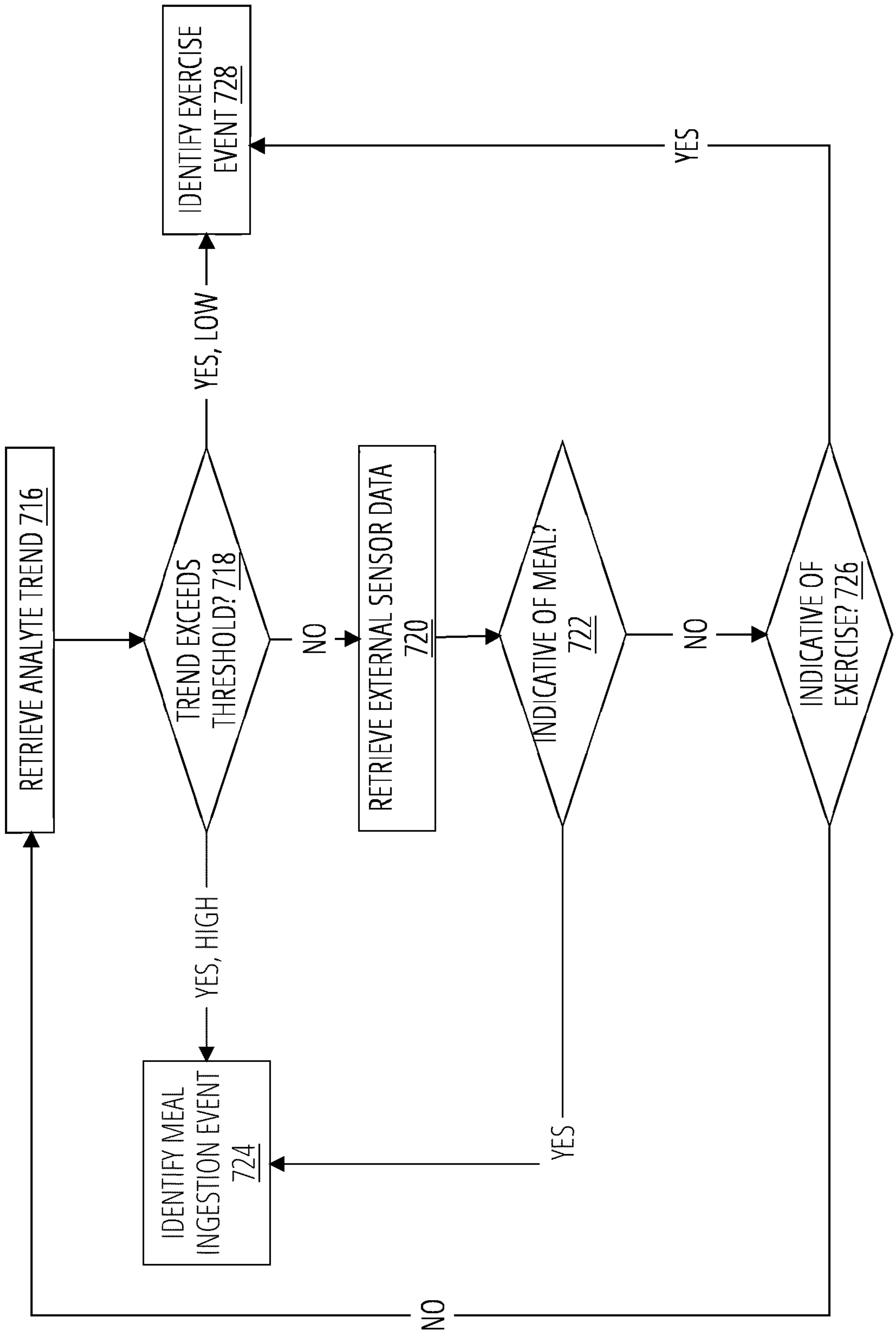
RETRIEVE ANALYTE TREND 716
TREND EXCEEDS THRESHOLD? 718
YES, LOW
YES, HIGH
NO
IDENTIFY EXERCISE EVENT 728
RETRIEVE EXTERNAL SENSOR DATA 720
INDICATIVE OF MEAL? 722
YES
NO
IDENTIFY MEAL INGESTION EVENT 724
INDICATIVE OF EXERCISE? 726
YES
NO

FIG. 7B

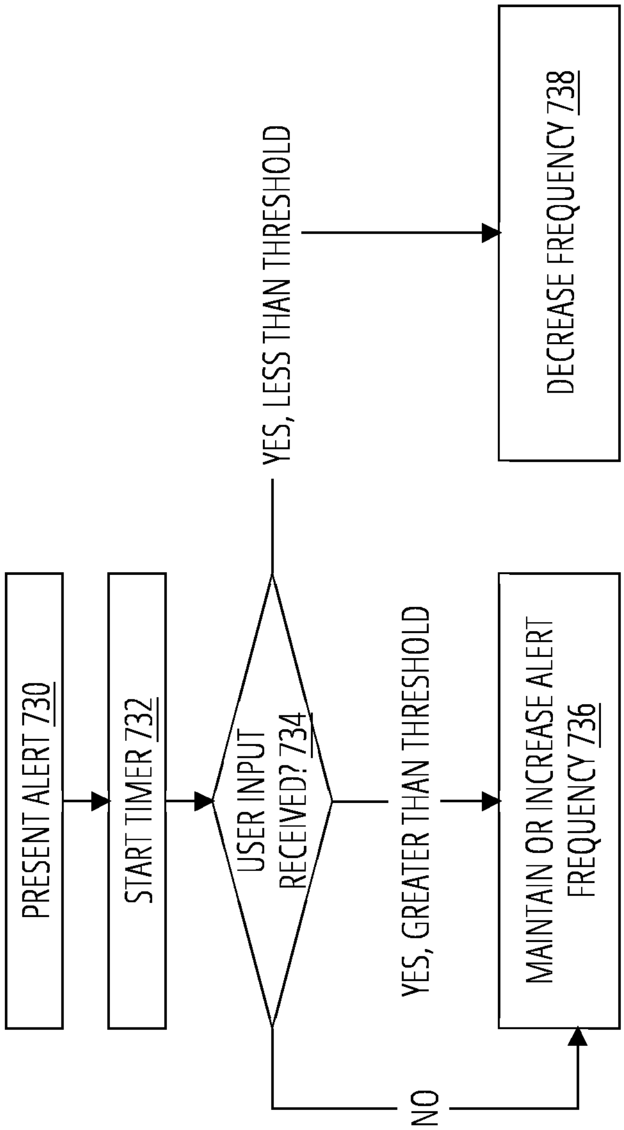
PRESENT ALERT 730
START TIMER 732
USER INPUT RECEIVED? 734
YES, LESS THAN THRESHOLD
DECREASE FREQUENCY 738
YES, GREATER THAN THRESHOLD
MAINTAIN OR INCREASE ALERT FREQUENCY 736
NO

FIG. 7C

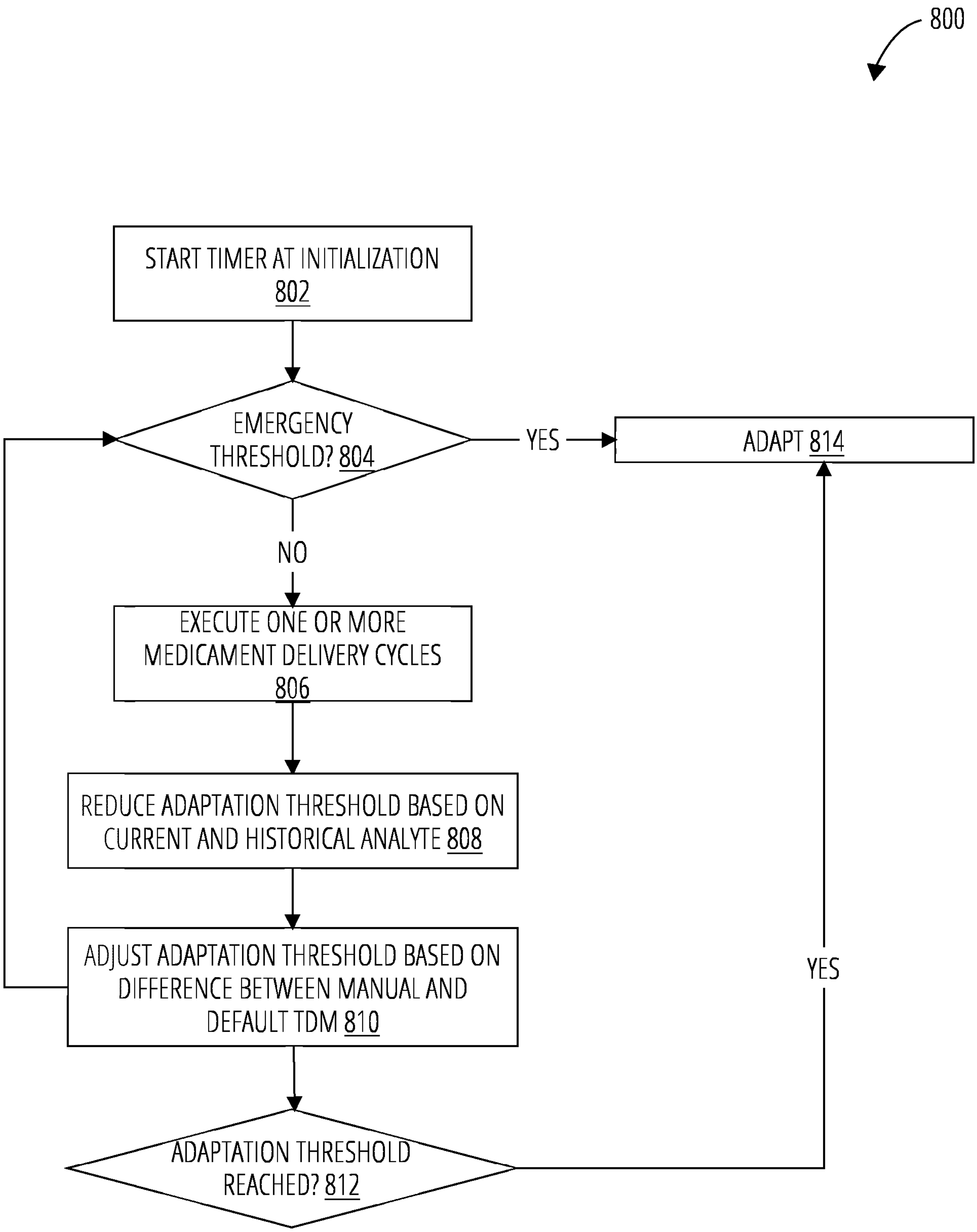
800
START TIMER AT INITIALIZATION 802
EMERGENCY THRESHOLD? 804
YES
ADAPT 814
NO
EXECUTE ONE OR MORE MEDICAMENT DELIVERY CYCLES 806
REDUCE ADAPTATION THRESHOLD BASED ON CURRENT AND HISTORICAL ANALYTE 808
ADJUST ADAPTATION THRESHOLD BASED ON DIFFERENCE BETWEEN MANUAL AND DEFAULT TDM 810
YES
ADAPTATION THRESHOLD REACHED? 812

FIG. 8

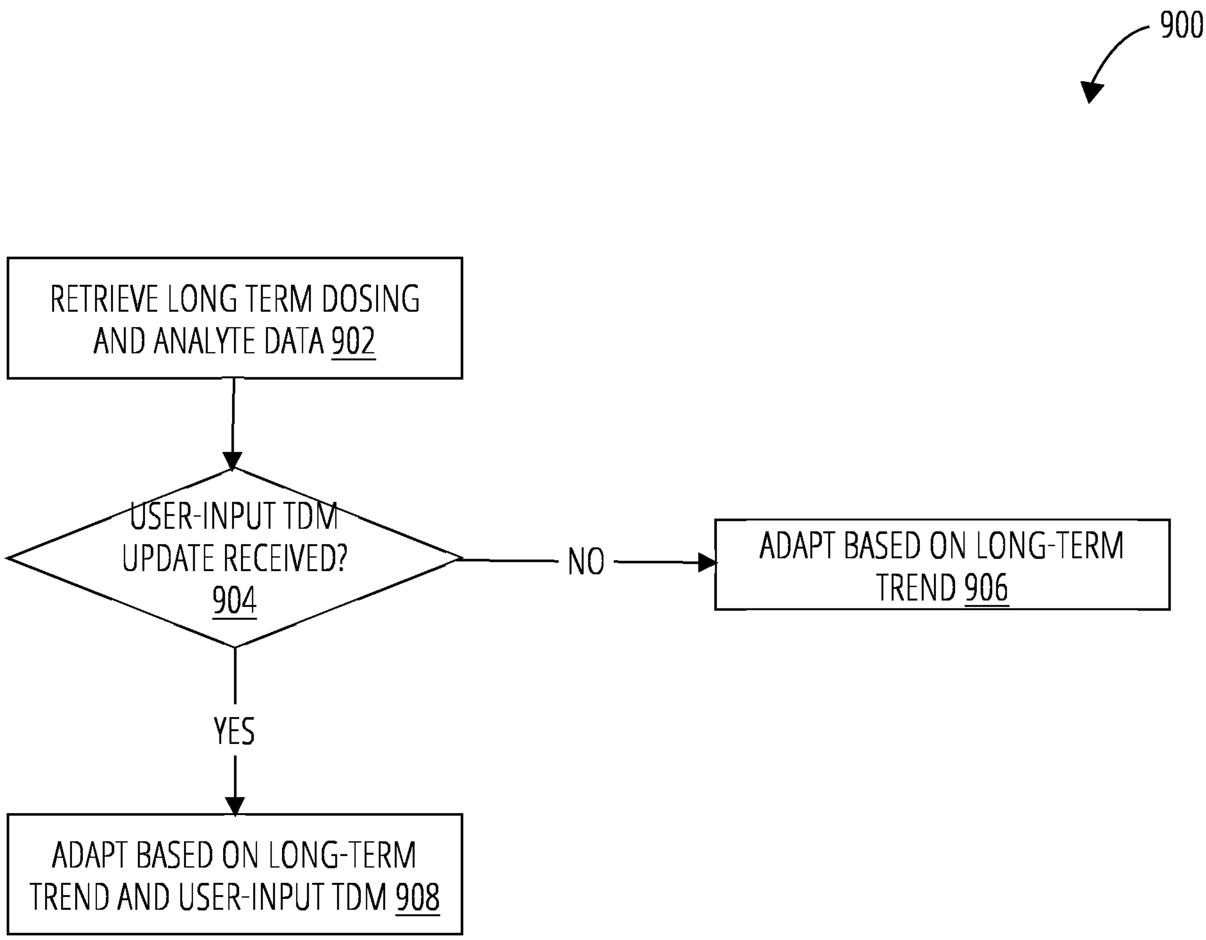
900
RETRIEVE LONG TERM DOSING AND ANALYTE DATA 902
USER-INPUT TDM UPDATE RECEIVED? 904
NO
ADAPT BASED ON LONG-TERM TREND 906
YES
ADAPT BASED ON LONG-TERM TREND AND USER-INPUT TDM 908

FIG. 9

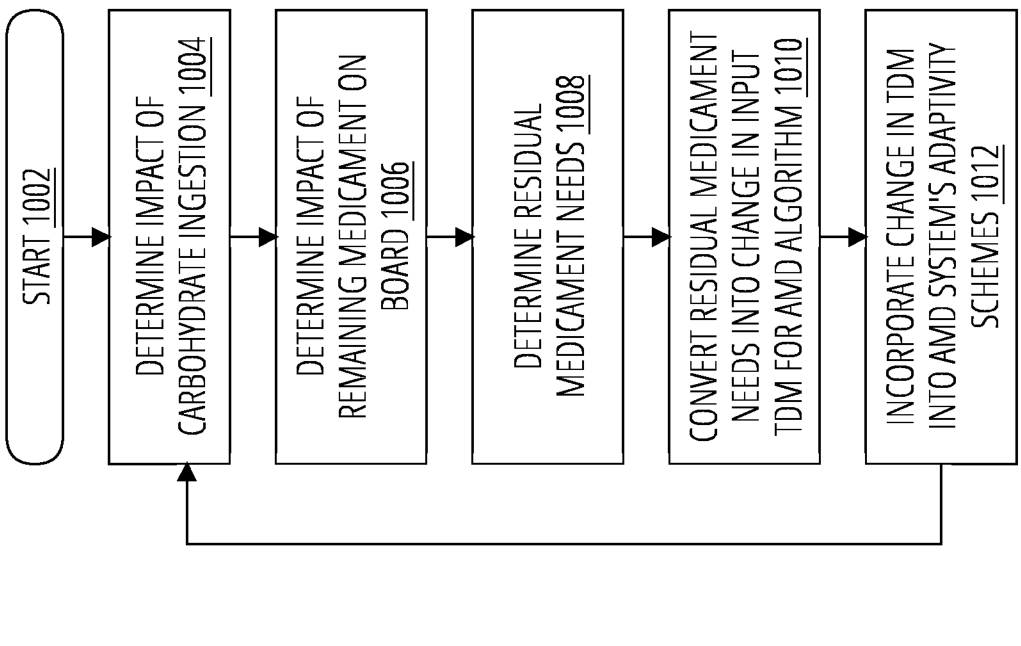


FIG. 10A

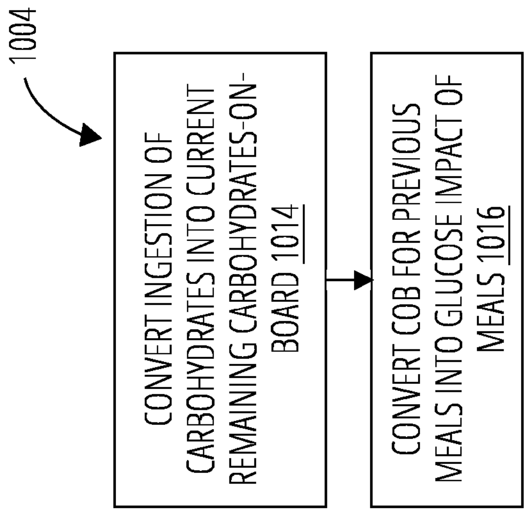


FIG. 10B

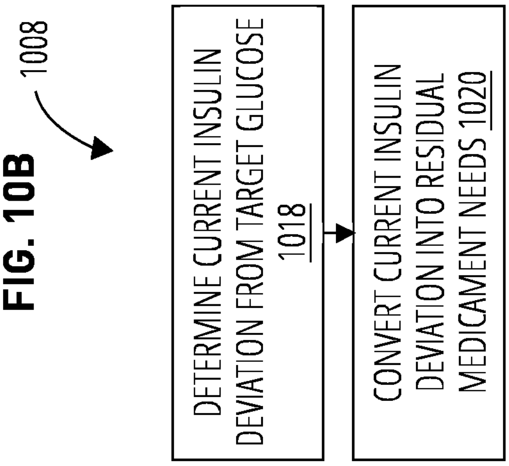


FIG. 10C

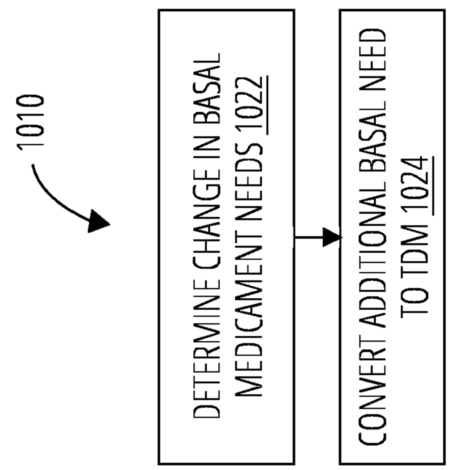


FIG. 10D

Europäisches Patentamt
European Patent Office
Office européen des brevets

# EUROPEAN SEARCH REPORT

Application Number
EP 25 18 7907

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/169332 A1 (SADEGHZADEH MAHTA [US] ET AL) 21 June 2018 (2018-06-21)<br>* abstract; figures 9,13 *<br>* paragraphs [0005], [0032], [0036], [0038], [0076], [0082], [0121] *<br>----- | 1-10 | INV.<br>G16H20/17<br>A61B5/145<br>A61B5/00<br>G16H40/63<br>G16H50/30<br><br>ADD.<br>A61M5/172<br>A61M5/142 |
| X | WO 2023/172637 A1 (INSULET CORP [US]) 14 September 2023 (2023-09-14)<br>* abstract *<br>* paragraphs [0010], [0012], [0019] *<br>----- | 1-10 | |
| X | WO 2024/081559 A1 (INSULET CORP [US]) 18 April 2024 (2024-04-18)<br>* abstract *<br>* paragraphs [0046] - [0050], [0054], [0055], [0059] *<br>----- | 1-10 | |
| X | WO 2023/141415 A1 (INSULET CORP [US]) 27 July 2023 (2023-07-27)<br>* abstract *<br>* paragraphs [0056] - [0060] *<br>----- | 1-10 | TECHNICAL FIELDS SEARCHED (IPC) |
| X | WO 2021/150442 A1 (INSULET CORP [US]) 29 July 2021 (2021-07-29)<br>* abstract *<br>* paragraphs [0004], [0016] - [0020] *<br>----- | 1-10 | G16H<br>A61B<br>A61M |

The present search report has been drawn up for all claims

1

| Place of search | Date of completion of the search | Examiner |
|---|---|---|
| Munich | 7 October 2025 | Ricciardi, Maurizio |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................................................
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 25 18 7907

This annex lists the patent family members relating to the patent documents cited in the above-mentioned European search report. The members are as contained in the European Patent Office EDP file on
The European Patent Office is in no way liable for these particulars which are merely given for the purpose of information.

07-10-2025

| Patent document cited in search report | Publication date | Patent family member(s) | Publication date |
|---|---|---|---|
| US 2018169332 A1 | 21-06-2018 | CA 3046100 A1 | 28-06-2018 |
| | | CN 110191731 A | 30-08-2019 |
| | | EP 3558422 A1 | 30-10-2019 |
| | | US 2018169332 A1 | 21-06-2018 |
| | | US 2018169333 A1 | 21-06-2018 |
| | | US 2018169334 A1 | 21-06-2018 |
| | | US 2018174675 A1 | 21-06-2018 |
| | | US 2021043297 A1 | 11-02-2021 |
| | | US 2021043298 A1 | 11-02-2021 |
| | | US 2021043299 A1 | 11-02-2021 |
| | | US 2021043300 A1 | 11-02-2021 |
| | | US 2022044784 A1 | 10-02-2022 |
| | | US 2023282328 A1 | 07-09-2023 |
| | | WO 2018119150 A1 | 28-06-2018 |
| WO 2023172637 A1 | 14-09-2023 | AU 2023231119 A1 | 29-08-2024 |
| | | EP 4490744 A1 | 15-01-2025 |
| | | JP 2025509229 A | 11-04-2025 |
| | | KR 20240157080 A | 31-10-2024 |
| | | US 2023285670 A1 | 14-09-2023 |
| | | WO 2023172637 A1 | 14-09-2023 |
| WO 2024081559 A1 | 18-04-2024 | EP 4602623 A1 | 20-08-2025 |
| | | US 2024123141 A1 | 18-04-2024 |
| | | WO 2024081559 A1 | 18-04-2024 |
| WO 2023141415 A1 | 27-07-2023 | AU 2023209422 A1 | 25-07-2024 |
| | | EP 4466711 A1 | 27-11-2024 |
| | | JP 2025502323 A | 24-01-2025 |
| | | US 2023230668 A1 | 20-07-2023 |
| | | WO 2023141415 A1 | 27-07-2023 |
| WO 2021150442 A1 | 29-07-2021 | EP 4094264 A1 | 30-11-2022 |
| | | US 2021228804 A1 | 29-07-2021 |
| | | WO 2021150442 A1 | 29-07-2021 |

EPO FORM P0459

For more details about this annex : see Official Journal of the European Patent Office, No. 12/82